(19) Europäisches Patentamt
European Patent Office
Office européen des brevets

(11) Publication number: **0 084 925**
**B1**

(12) **EUROPEAN PATENT SPECIFICATION**

(45) Date of publication of patent specification: **09.04.86**

(21) Application number: **83300042.5**

(22) Date of filing: **05.01.83**

(51) Int. Cl.⁴: **C 07 D 499/00,**
C 07 D 499/32, A 61 K 31/43,
A 61 K 31/545, C 07 D 519/00
// (C07D519/00,
499:00),(A61K31/545, 31:43)

# (54) 6-Aminoalkylpenicillanic acid 1,1-dioxides and derivatives as beta-lactamase inhibitors.

(30) Priority: **11.01.82 US 338797**
**14.06.82 US 388324**
**14.06.82 US 388323**
**21.10.82 US 434371**

(43) Date of publication of application:
**03.08.83 Bulletin 83/31**

(45) Publication of the grant of the patent:
**09.04.86 Bulletin 86/15**

(84) Designated Contracting States:
**AT BE CH DE FR GB IT LI LU NL SE**

(56) References cited:
**GB-A-2 053 220**
**US-A-4 237 051**

**Tetrahedron Letters No. 9, pp. 381-385, 1962**

(73) Proprietor: **PFIZER INC.**
**235 East 42nd Street**
**New York, N.Y. 10017 (US)**

(72) Inventor: **Barth, Wayne Ernest**
**40 Monticello Drive East Lyme**
**New London Connecticut (US)**

(74) Representative: **Moore, James William, Dr.**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 084 925 B1

Courier Press, Leamington Spa, England.

**Description**

The present invention relates to 6-alpha- and 6-beta-(aminomethyl and 1-aminoethyl)penicillanic acid 1,1-dioxides, said aminomethyl compounds opitonally substituted on amino nitrogen with benzyl, hydroxybenzyl, picolyl or phenethyl, pharmaceutically-acceptable salts thereof and conventional esters thereof which are hydrolyzable *in vivo; bis*-methanediol esters thereof; or mixed methanediol esters with said beta-lactamase inhibitors and sulbactam (penicillanic acid 1,1-dioxide), said methanediol esters also hyrolyzable *in vivo.* While some of these compounds possess antibacterial activity *per se,* their principal value is as beta-lactamase inhibitors. Thus they are useful in combination with conventional beta-lactam antibiotics (penicillins and cephalosporins) against microorganisms resistant or partially resistant to beta-lactam antibiotics through production of beta-lactamase enzymes. Also encompassed by the present invention are pharmaceutical compositions comprising a present beta-lactamase inhibiting compound and a known beta-lactam antibiotic; *bis*-methanediol esters of the present beta-lactamase inhibiting compounds with either ampicillin or amoxicillin; pharmaceutical compositions of said *bis*-esters; and compounds useful as intermediates in the preparation of these compounds.

Related compounds, *viz,* penicillanic acid, 1,1-dioxide and esters thereof readily hydrolyzable *in vivo* (Barth, U.S. Patent 4,234,579); the *bis*-methanediol ester of sulbactam (Bigham, U.S. Patent 4,309,347); various 6-beta-(hydroxymethyl)penicillanic acid, 1,1-dioxides and esters thereof (Kellogg, U.S. Patent 4,287,181); and 6-beta-(aminomethyl)penicillanic acid (McCombie, U.S. Patent 4,237,051) have been previously described as beta-lactamase inhibitors useful in combination with beta-lactam antibiotics for the treatment of bacterial infections. Antibacterial *bis*-esters of methanediol with penicillins and penicillanic acid 1,1-dioxide (Bigham, U.S. Patent 4,244,951; Godtfredsen *et al.,* U.S. Patent 4,342,772) have also been described.

U.K. Patent Application 2,053,220, published February 4, 1981, broadly discloses beta-lactamase inhibiting compounds of the formula

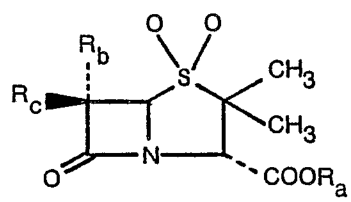

The definitions of $R_a$, $R_b$ and $R_c$ define literally an infinite number of compounds. These definitions, by appropriate selection of $R_a$, $R_b$ and $R_c$, may possibly define the simple 6-alpha and 6-beta-(aminoalkyl)-penicillanic acid 1,1-dioxides of the present invention. No specific method for preparation of these compounds is present in the disclosure of this U.K. application, and there is no hint or suggestion that from among the infinity of compounds proposed, the present aminomethyl and 1-aminoethyl compounds are preferred compounds, possessing the particularly highly potent beta-lactamase inhibitory activity which we have determined for them.

The present invention relates to compounds of the formulae

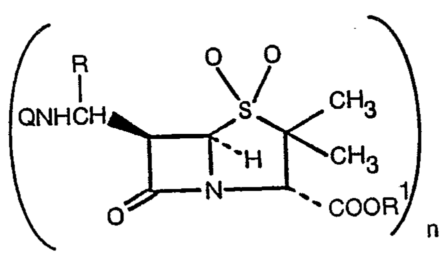 (I) and 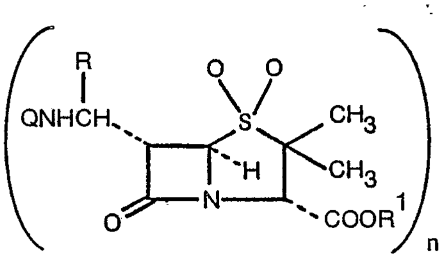 (II)

wherein in a first alternative:

R is hydrogen;
n is 1;
Q is benzyl, *o-, m-* or *-p*-hydroxybenzyl, phenethyl, or 2-, 3- or 4-picolyl; and
$R^1$ is hydrogen, or a radical group forming an ester which is hydrolyzable under physiological conditions;

in a second alternative:
Q is hydrogen;
R is hydrogen or methyl; and
n is 1 and

$R^1$ is hydrogen, a radical group forming an ester hydrolyzable under physiological conditions, or 1,1-dioxopenicillanoyloxymethyl; or

n is 2

and $R^1$ is $—CH_3—$; the pharmaceutically-acceptable acid addition salts thereof; and the pharmaceutically-acceptable cationic salts thereof when $R^1$ is hydrogen.

It will be understood by those skilled in the art that when R is methyl, each of the formulae (I) and (II) represent two different diastereoisomers (epimers). Depending on absolute stereochemistry, the side chains of these epimeric pairs are designated 1R-aminoethyl and 1S-aminoethyl. In each case one epimer has side chain configuration R and the other S. The same is true with respect to the formulae (III) and (IV), (V) and (VI), and (VII) and (VIII) below. These isomer pairs are generally separable by column chromatography at a variety of stages in the overall processes which are detailed below.

Pharmaceutically-acceptable acid addition salts include, but are not limited to, those with hydrochloric acid, sufuric acid, nitric acid, phosphoric acid, citric acid, maleic acid, succinic acid, benzene-sulfonic acid, p-toluenesulfonic acid, 2-naphthlenesufonic acid and methanesulfonic acid. Pharmaceutically-acceptable cationic salts include, but are not limited to, those of sodium, potassium, calcium, N,N'-dibenzylethylendiamine, N-methylglucamine (meglumine) and diethanolamine.

The reference to esters which are hydrolyzable under physiological conditions refers to those esters frequently referred to as "pro-drugs". Such esters are now as well-known and common in the penicillin art as pharmaceutically-acceptable salts. Such esters are generally used to enhance oral absorption, but in any event are readily hydrolyzed *in vivo* to the parent acid. Preferred esters show no tendency to hydrogenolyze under the conditions preferably employed for their preparation (see below). The more preferred ester forming radicals are:

gamma-butyrolacton-4-yl,
$—CHR^2OCOR^3$, and
$—CHR^2OCOOR^3$,

wherein

$R^2$ is hydrogen or methyl and
$R^3$ is $(C_1—C_6)$alkyl. The most preferred radicals are pivaloyloxymethyl and 1-ethoxycarbonyloxyethyl.
When n is 1 and $R^1$ is 1,1-dioxopenicillanogloxymethyl,

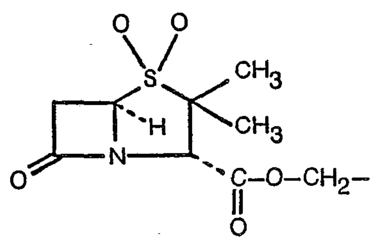

the compounds of the formulae (I) and (II) are diesters of methanediol. SUch esters are also hydrolyzable under physiological conditions, yielding the parent acid of the formula (I) or (II), wherein n is 1 and $R^1$ is H, and pencillanic acid 1,1-dioxide. The latter compound also possesses beta-lactamase inhibitory activity. When n is 2 and $R^1$ is $—CH_2—$, the *bis*-ester is likewise hydrolyzed under physiological conditions, now producing two molecules of the parent acid from each molecule of the *bis*-ester.

Both the 6-beta-compounds (I) and the 6-alpha-compounds (II), regardless of side chain stereochemistry when R is methyl, are potent beta-lactamase inhibitors. For oral use, it is preferred that $R^1$ is other than hydrogen. When $R^1$ is a radical group forming an ester hydrolyzed *in vivo*, the preferred radicals are defined above. Because it is readily prepared in a highly pure, crystalline state (directly useful in mammals as a pharmaceutically-acceptable salt) pivaloyloxymethyl 6-alpha-(aminoethyl)penicillanate 1,1-dioxide *p*-toluenesulfonate salt is a particularly valuable compound in this series. Also readily prepared and of special value for oral use are the 6-alpha(aminomethyl) derivatives (II) wherein n is 1 and $R^1$ is 1,1-dioxopencillanoyloxymethyl or n is 2 and $R^1$ is $—CH_2—$.

Both the 6-beta-compounds (I) and the 6-alpha-compounds (II), regardless of side chain stereochemistry when R is methyl, are potent beta-lactamase inhibitors.

The compounds of the formulae (I) and (II) are useful as inhibitors of beta-lactamase enzymes. By this mechanism, these compounds enhance the activity of beta-lactam antibiotics (penicillins and cephalosporins), particularly against those microorganisms which are resistant or partially resistant to the beta-lactam antibiotic through the production of enzymes (beta-lactamases) which would otherwise destroy or partially destroy the beta-lactam antibiotic. In this manner, the spectrum of activity of the beta-lactam antibiotic is increased.

The beta-lactam antibiotics are one of the most well-known and widely-used class of antibacterial agents. These compounds are characterized by a nucleus consisting of a 2-azetidinone (beta-lactam) ring fused to either a thiazolidine or a dihydro-1,3-thiazine ring. When the nucleus contains a thiazolidine ring,

the compounds are usually referred to generically as penicillins, whereas when the nucleus contains a dihydrothiazine ring, the compounds are referred to as cephalosporins. While the present compounds are effective in enhancing the activity of beta-lactam antibiotics in general, their preferred use is found in their combination with a penicillin or cephalosporin of established clinical utility, *viz.,* amoxicillin, ampicillin, azlocillin, bacampicillin, carbenicillin, carbenicillin indanyl, carbenicillin phenyl, cefaclor, cefadroxil, cefaloram, cefamandole, cefamandole nafate, cefaparole, cefatrizine, cefazolin, cefonicid, cefmenoxime, cefoxizime, cefoperazone, ceforanide, cefotaxime, cefoxitin, cefsulodin, ceftazidime, ceftizoxime, ceftriaxone, cefuroxime, cephacetrile, cephalexin, cephaloglycin, cephaloridine, cephalothin, cephapirin, cephradine, cyclacillin, epicillin, hetacillin, levopropylcillin, mecillinam, mezlocillin, penicillin G, penicillin V, phenethicillin, piperacillin, pirbenicillin, pivampicillin, sarmoxicillin, sarpicillin, suncillin, talampicillin and ticarcillin, , including the pharmaceutically-acceptable salts thereof. The names employed for these beta-lactams are generally USAN, i.e., United States Adopted Names. Preferred combinations are with ampicillin or an ampicillin derivative, with amoxicillin or an amoxicillin derivative or, most particularly with cefoperazone.

Although the compounds of the present invention can be administered separately from the beta-lactam antibiotic, combination dosage forms are preferred. The pharmaceutical composition, whether for oral or parenteral use, comprises in a ratio of 1:3 to 3:1 by weight a beta-lactamase inhibitor of the formula (I) or (II) and a beta-lactam antibiotic, in total amounts sufficient to successfully treat a bacterial infection in a mammal in a single or, more usually, multiple doses.

Also encompassed by the present invention are antibacterial compounds having the stereochemical formulae

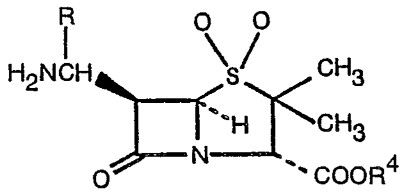 (III) and 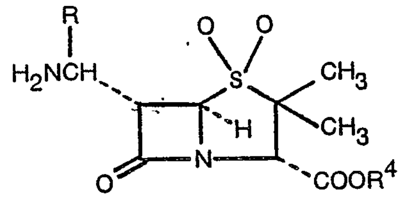 (IV)

wherein

R is hydrogen or methyl; and

$R^4$ is

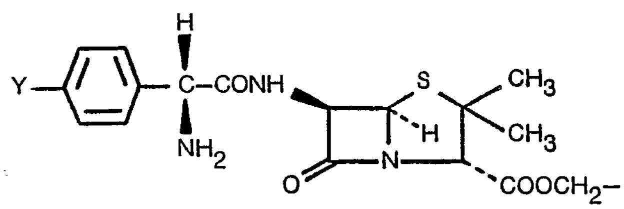

wherein Y is hydrogen, hydroxy, ($C_2$—$C_7$)-alkanoyloxy, ($C_2$—$C_7$)-alkoxycarbonyloxy, benzoyloxy, or benzoyloxy monosubstituted with ($C_1$—$C_4$)-alkyl, ($C_1$—$_4$)alkoxy or halo; and the pharmaceutically-acceptable mono- and diacid addition salts thereof, the acids being as described above.

These *bis*-methanediol esters (III) and (IV) are effective as antibacterials through their in vivo, hydrolysis to the corresponding beta-lactamase inhibitors of the formulae (I) and (II) and to the corresponding ampicillin, amoxicillin or amoxicillin substituted on phenolic oxygen. It will be further noted that such phenolic esters are also generally hydrolyzed *in vivo* to produce amoxicillin. The *bis*-methanediol ester compounds are formulated in to pharmaceutical compositions, suitable for either parenteral or oral administration in single or (more usually) multiple dosage for the treatment of bacterial infections in mammals.

Preferred compounds of the formulae (III) and (IV) have Y as hydrogen or hydroxy; most preferred compounds have Y as hydrogen.

Further encompassed by the present invention are intermediates of the stereochemical formulae

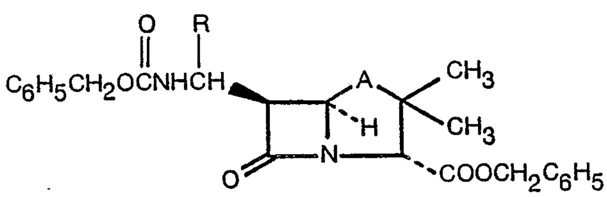 , (V)

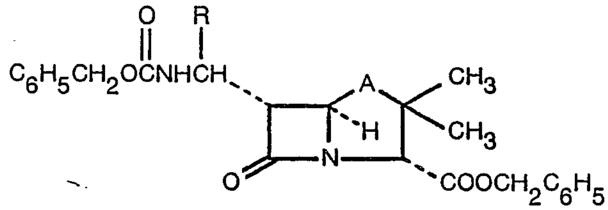

(VI)

wherein

A is

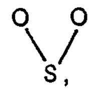, 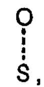, 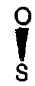 or S;

and

R is hydrogen or methyl;

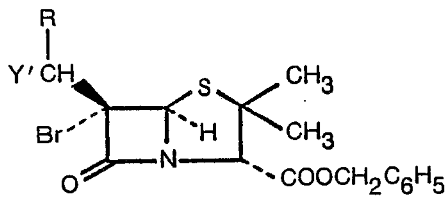

(VII) and

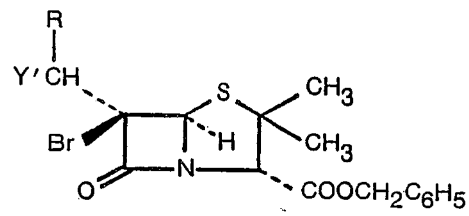

(VIII)

wherein

Y' is benzloxycarbonylamino, amino, azido or trifluoromethanesulfonyloxy; and

R is hydrogen or methyl.

Those compounds of the present invention of the formulae (I) and (II) are generally prepared from benzyl 6,6-dibromopenicillanate or from benzyl 6-alpha-iodopenicillanate.

A preferred route, particularly for the 6-beta-(aminomethyl) series, involves as a first stage conversion of the dibromo compound to an epimeric mixture of mono-Grignard reagents. This is conveniently done by an exchange reaction using essentially one molar equivalent of methyl magnesium bromide in an ether solvent (ether, tetrahydrofuran, dimethoxyethane) at low temperature (−50°C to −100°C), conveniently at −78°C, the temperature of an acetone-dry ice bath. After a brief reaction time (5—30 minutes) at such reduced temperature, the mono-Grignard reagents are contacted with essentially 0.5 molar equivalents of benzyloxycarboxamidomethyl acetate (usually diluted with the same ether solvent and added to the cold Grignard reagent at such a rate that the low temperature of the reaction is maintained). Reaction time is not critical; 0.5 to 2 hours at −50° to −100°C is usually sufficient to achieve complete reaction. Mixed epimers of the above formulae (VII) and (VIII) wherein Y' is benzyloxycarbonylamino are readily recovered by acetic acid quench, concentration and chromatography. The mixture of epimers can be used directly in the next step, or if desired, separated by further column chromatography on silica gel.

The next step of the sequence is reductive removal of the bromine atom, conveniently accomplished by the action of excess tri-*n*-butyltin hydride, optionally in the presence of small amounts (less than 0.1 molar equivalents) of a free radical initiator such as 2,2'-azo*bis*isobutyronitrile (AIBN). Here and hereinafter, "reaction-inert solvent" is defined as a solvent which does not react with starting materials, reagents, intermediates or products in manner which significantly reduces the yield of the desired product. Well-suited in the present case are hydrocarbon solvents such as benzene or toluene. Temperature should be elevated (60—100°C), such that reaction occurs in a reasonable time, but not so high as to cause undue thermal degradation. When this step is carried out on the mixed epimer precursors, the 6-beta-epimer (V), wherein A is S, is generally recovered by crystallization; if desired the 6-alpha-epimer (VI, A = S) is recovered from the mother liquors by evaporation and chromatography.

A second preferred route, particularly for the 6-alpha(aminomethyl) series, is to react a cold ether solution of the Grignard reagent from benzyl 6-alphaiodopenicillanate with a benzyloxycarboxamidomethyl acetate under conditions described above. The resulting mixture of compounds (V) and (VI), wherein A is S, can be separated by column chromatography, but preferably are oxidized to 1,1-dioxides and then subjected to C—6 epimerization conditions to yield the clean alpha-epimer (VI, A = $SO_2$) as detailed below.

To form the 1-alpha and 1-beta oxides of the formulae (V) and (VI), where A is S----O or S◀O, the above sulfides of the formulae (V) and (VI), wherein A is S, are oxidized with substantially 1 molar equivalent of a peracid, conveniently *m*-chloroperbenzoic acid, in a reaction-inert solvent such as ethyl acetate at 0°—50°C. When benzyl 6-beta-(benzyloxycarbonylaminomethyl)penicillanate (V, R = H, A = S) is oxidized, the resulting alpha-oxide (V, R = H, A = S◀O) is isolated by crystallization, while the beta-oxide (V, R = H, A = S◀O) is isolated from mother liquors by evaporation. If desired, other standard

sulfoxide forming reagents can be used.

If desired, benzyl 6-beta-(benzyloxycarbonylaminomethyl)penicillanate 1-beta-oxide (V, $A = S \rightarrow O$) is rearranged to the corresponding 6-alpha epimer (VI, $A = S \rightarrow O$) by contacting the former with an equivalent of 1,5-diazabicyclo[4.3.0]non-5-ene (DBN) at 0—50°C, conveniently at 25° where reaction is complete in a very short time (3—15 minutes). In like manner a 6-beta dioxide (V, $A = SO_2$) is converted to its 6-alipha epimer (VI, $A = SO_2$).

Oxidation of the sulfides (V) and (VI), wherein A is S, or further oxidation of the above sulfoxides, with excess peracid (but otherwise under conditions as generally described above with mono-oxide formation) yields the corresponding sulfones (1,1-dioxides) of the formulae (V) and (VI), wherein A is $SO_2$. If desired, other sulfone forming reagents such as $KMnO_4$ can be used.

Hydrogenolyis of the resulting benzyl 6-(alpha or beta) (benzyloxycarbonylaminoalkyl)penicillanate 1,1-dioxides, (V and VI, $A = SO_2$) produces the corresponding 6-(alpha or beta) (aminomethyl or 1-aminoethyl)penicillanic acids (I and II, $Q = R^1 = H$). Hydrogenolysis is carried out by methods well-known in the penicillin art. The substrate, in a reaction-inert solvent, is contacted with hydrogen in the presence of a noble metal catalyst, such as palladium, platinum or rhodium, optionally in the form of its oxide or a salt, or on a carrier such as carbon, an alkaline earth carbonate or alumina. Temperature is not critical (e.g. 0—50°C), but is preferably 25°C or lower in order to minimize thermal degradation. Pressure can be varied over a wide range (subatmospheric to 100 atmospheres), but as a matter of convenience will generally be in the range of 1 to 7 atmospheres. The reaction inert solvent is preferably relatively low boiling so as to be readily removed by concentration *in vacuo.* Aqueous tetrahydrofuran is a solvent particularly well-suited for the present purpose. The preferred catalyst is palladium, supported on carbon.

To prepare an *in vivo* hydrolyzable ester [i.e., a compound of the formulae (I) or (II), wherein Q is hydrogen and $R^1$ is a radical group forming an ester which is hydrolyzable under physiological conditions], the amino group of the 6-(aminoalkyl)penicillanic acid 1,1-dioxide is first protected with a benzyloxycarbonyl group, using methods well-known in the art. For example, benzyl chloroformate is added slowly to the amine in a reaction-inert solvent such as aqueous acetone or aqueous tetrahydrofuran while maintaining pH 8.0 at a temperature of 0—35°C, preferably 0—20°C. In this manner, compounds of the formulae (IX) and (X), wherein $R^1$ is H, are formed. Alternatively, such compounds are formed by the partial hydrogenolysis of compounds of the formula (V) and (VI) wherein A is $So_2$. Except to limit hydrogen uptake, conditions as described above are used.

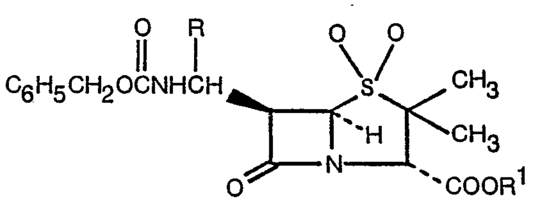

(IX)

and

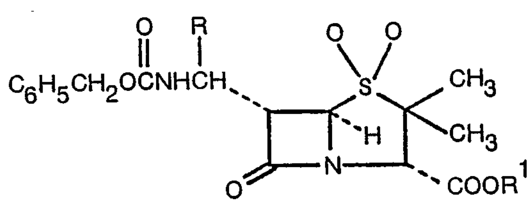

(X)

The latter intermediates are then converted to the desired esters of the formulae (IX) and (X), wherein $R^1$ now represents an *in vivo* hydrolyzable ester, according to known methods, readily identified by those skilled in the penicillin art (see for example U.S. Patents 4,234,579 and 4,287,181; and European patent publication No. 40,494). Preferred ester values of $R^1$ have been defined above; preferred methods for the preparation of such esters are detailed in specific examples below and in European patent publication No. 40,494.

The protected esters (IX) and (X) are converted to the desired esters of the formula (I) or (II) wherein Q is hydrogen, retaining $R^1$ as the ester functionality, by hydrogenolysis, preferably in the presence of a weakly acidic buffer comprising equimolar quantities of a weakly basic amine, such as pyridine, and a strong acid such as mineral acid (e.g. HCl, $HNO_3$, $H_2SO_4$) or preferably a sulfonic acid (such as methanesulfonic acid, benzenesulfonic acid or *p*-toluenesulfonic acid), otherwise according to methods described above, taking care to minimize exposure to conditions (e.g. water, lower alcohols, high acidity or basicity) which will cause hydrolysis of the sensitive ester or beta-lactam groups. It is preferred to isolate the ester directly from the reaction mixture in the form of the acid addition salt where the acid is the one used in the buffer. A particularly preferred buffer is pyridinium *p*-toluenesulfonate in which case the product is usually isolated as its *p*-toluenesulfonate salt.

The compounds of the formulae (I) and (II) in the first alternative (i.e., wherein n is 1, R is hydrogen and Q is other than hydrogen) are readily prepared by reductive alkylation of the simple 6-(aminomethyl) compounds of the above formulae (I) and (II) wherein R and Q are both hydrogen, in the presence of an equimolar amount of the corresponding aldehyde, using sodium cyanoborohydride as the reducing agent. One molar equivalent of the aldehyde is fully satisfactory; an excess of sodium cyanoborohydride is generally used, e.g., about two thirds of a mole per mole of substrate. Temperature is not critical and can be in the range of 0—50°C; conveniently ambient temperature is employed.

The above-defined pharmaceutically-acceptable acid addition salts of the present invention are readily prepared by standard methods. For example, and equivalent of the acid is combined with the free amine form of the compound in an organic or aqueous organic solvent. The salt is isolated by concentration and/or the addition of a non-solvent. In some cases, the salt is isolated directly from a reaction mixture, without isolation of the free amine.

The above-defined pharmaceutically-acceptable cationic salts of those compounds of the present invention having a free carboxylic acid group are also readily prepared by standard methods. For example, an equivalent of the corresponding cationic hydroxide, carbonate or bicarbonate or of an amine is combined with the carboxylic acid in an organic or aqueous solvent, preferably at reduced temperature (e.g. 0—5°C), with vigorous agitation and slow addition of the base. The salt is isolated by concentration and/or the addition of a non-solvent. In some cases, the salt is isolated directly from a reaction mixture, without isolation of the free acid form.

The *bis*-methanediol esters (III) and (IV), as well as those of the formulae (I) and (II) wherein n is 1 and $R^1$ is 1,1-dioxopenicillanoyloxymethyl or n is 2 and $R^1$ is $—CH_2—$, are also prepared from the amino protected penicillanic acid acids (IX) and (X), wherein $R^1$ is H. In one alternative, the latter compounds are first converted to the corresponding chloromethyl esters. The preferred method is to convert the acid to its tetrabutylammonium salt, which is then reacted with excess chloromethyl iodide at 0—50°C, preferably at 25° or less.

Although a chloromethyl ester can be used directly in the next step, it is preferred to first convert the chloromethyl ester to the corresponding iodomethyl ester. Contact of the chloromethyl ester with sodium iodide in acetone at 0—50° until reaction is substantially complete represents conditions particularly well-suited to this purpose.

The iodomethylester is then reacted, in a reaction inert solvent at 0—50°C, with a salt of penicillanic acid 1,1-dioxide; a salt of the same amino protected penicillanic acid (IX) or (X) wherein $R^1$ is H; a salt of azidocillin [6-(D-2-phenyl-2-azidoacetamido)penicillanic acid]; or a salt of an ampicillin or amoxicillin derivative of the formula

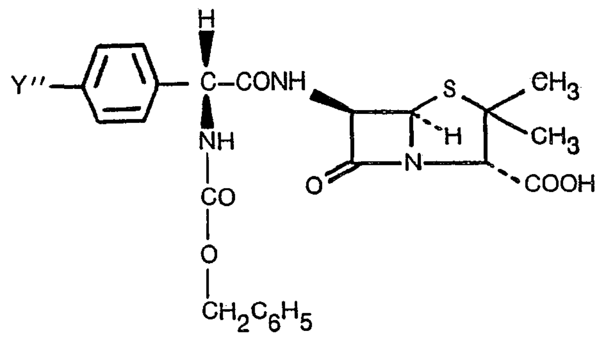

(XI)

wherein Y'' is H, benzyloxycarbonyloxy, ($C_2$—$C_7$)alkoxycarbonyloxy, benzoyloxy, or benzoyloxy monosubstituted with ($C_1$—$C_4$)alkyl, ($C_1$—$C_4$)alkoxy or halo (F, Cl or Br). The preferred salt is the tetrabutylammonium salt, since it reacts very rapidly with the iodomethyl ester, minimizing degradation.

In a second alternative, the *bis*-methanediol esters (III) and (IV), as well as the esters of the formula (I) or (II) where n is 1 and $R^1$ is 1,1-dioxopenicillanoyloxymethyl are prepared by reacting the above salt of an amino-protected penicillanic acid (IX) or (X), wherein $R^1$ is H, with a halomethyl ester (preferably and iodomethyl ester) of penicillanic acid 1,1-dioxide, of azidocillin, or of an amplicillin or amoxicillin derivative of the formula (XI) above.

In either alternative, the resulting protected methanediol diester is then converted to the desired end product of the formula (I), (II) [where n is 1 and $R^1$ is 1,1-dioxopenicillnoyloxymethyl or n is 2 and $R^1$ is $—CH_2—$], (III) or (IV) by hydrogenolysis, using methods detailed above; again minimizing exposure to conditions which cleave the sensitive methanediol ester bonds. The pharmaceutically-acceptable mono- or diacid addition salts of these methanediol diesters are prepared using one or two equivalents of the acid, as appropriate, according to methods described above.

Another preferred route to the intermediates of the formulae (VII) and (VIII) wherein Y' is benzyloxycarbonylamino, particularly when R is methyl, is to prepare them from the corresponding known benzyl 6-alpha-(hydroxymethyl or 1-hydroxyethyl)-6-beta-bromopenicillanate and benzyl 6-beta-(hydroxymethyl or 1-hydroxyethyl)-6-alpha-bromopenicillanate (also prepared form benzyl 6,6-dibromopenicillanate).

In the first stage, the above hydroxymethyl or 1-hydroxyethyl compounds are converted to the corresponding trifluoromethanesulfonate esters (VII and VIII, Y' = trifluoromethanesulfonyloxy). This reaction is conveniently carried out at room temperature using trifluoromethanesulfonic anhydride as reagent, in a reaction inert solvent such a methylene chloride in the presence of at least one equivalent of a *tert*-amine such as pyridine or diisopropylethylamine.

In the second stage the sulfonate group is displaced by azide, forming the azidomethyl compounds of the formulae (VII) and (VIII), wherein Y' is azido. An excellent reagent for this purpose is tetramethylguanidinium azide in moderate excess. The reaction is carried out at 0—25°C, preferably about 10°C, in a reaction-inert solvent such as chloroform or methylene chloride.

In the third stage the azido group is reduced to an amino group, yielding compounds of the formulae (VII) and (VIII) wherein Y' is amino. A convenient reagent for this purpose is hydrogen sulfide, in the presence of a tertiary amine such as triethylamine in a reaction-inert solvent such as chloroform. Gaseous hydrogen sulfide is bubbled through the reaction mixture at 0—50°C until reduction is substantially complete (usually about 3—4 hours at 25°C).

Finally, the amino group is protected with a benzyloxycarbonyl group, using conditions standard in the art. For example benzyl chloroformate as reagent in the presence of a tertiary amine such as pydridine or N,N-diisopropylethyl amine, in a reaction-inert solvent such as methylene chloride at 0—50°C, preferably at reduced temperature (0—10°C). The resulting compounds of the formulae (VII) and (VIII) wherein Y' is benzyloxycarbonylamino are then further processed according to methods detailed above.

As indicated above, some of the compounds of the formulae (I) and (II), generally those wherein $R^1$ is hydrogen, have *in vitro* antibacterial activity. Such activity is demonstrated by measuring the minimum inhibitory concentrations (MIC's) in mcg/ml against a variety of microorganisms. The procedure which is followed is the one recommended by the International Collaborative Study on Antibiotic Sensitivity Testing (Ericcson and Sherris, *Acta. Pathologica et Microbiologia Scandinav,* Supp. 217, Section B: 64—68 [1971]), and employs brain heart infusion (BHI) agar and the inocula replicating device. Overnight growth tubes are diluted 100 fold for use as the standard inoculum (20,000—10,000 cells in approximately 0.002 ml are placed on the agar surface; 20 ml of BHI agar/dish). Twelve 2 fold dilutions of the test compound are employed, with initial concentration of the test drug being 200 mcg/ml. Single colonies are disregarded when reading plates after 18 hours at 37°C. The susceptibility (MIC) of the test organism is accepted as the lowest concentration of compound capable of producing complete inhibition of growth as judged by the naked eye.

Those compounds of the formulae (I) and (II) having said *in vitro* antibacterial activity are thus useful as industrial antimicrobials, for example in water treatment, slime control, paint preservation and wood preservation, as well as for topical application as disinfectant. In the case of use of these compounds for topical application, it is often convenient to admix the active ingredient with a non-toxic carrier, such as vegetable or mineral oil or an emollient cream. Similarly, it can be dissolved or dispersed in liquid diluents or solvents such as water, alkanols, glycols or mixtures thereof. In most instances it is appropriate to employ concentrations of the active ingredient of from about 0.1 percent to about 10 percent by weight, based on total composition.

As also indicated above, the compounds of the formulae (I) and (II) are of more particular value as potent inhibitors of microbial beta-lactamases. By this mechanism they increase the antibacterial effectiveness of beta-lactam antibiotics (penicillins and cephalosporins) against many microorganisms, particularly those which produce a beta-lactamase. The ability of the said compounds of the formula (I) or (II) to increase the effectiveness of a beta-lactam antibiotic can be appreciated by reference to experiments in which the MIC values of the antibiotic alone, and compound of the formula (I) or (II) (having $R^1$ as hydrogen) alone, are determined. These MIC's are then compared with the MIC values obtained with a combination of the given antibiotic and the compound for the formula (I) or (II), wherein $R^1$ is hydrogen. When the antibacterial potency of the combination is significantly greater than would have been predicted from the potencies of the individual compounds, this is considered to constitute enhancmenet of activity. The MIC values of combinations are measured using the method described by Barry and Sabath in "Manual of Clinical Microbiology", edited by Lenette, Spaulding and Truant, 2nd Edition, 1974, American Society for Microbiology.

The compounds of the formulae (I) and (II) enhance the antibacterial effectiveness of beta-lactam antibiotics *in vivo.* That is, they lower the amount of the antibiotic which is needed to protect mice against an otherwise lethal inoculum of certain beta-lactamase producing bacteria. In determining such activity, acute experimental infections are produced in mice by the intraperitoneal inoculation of the mice with a standardized culture of the test organism suspended in 5 percent hog gastric mucin. Infection severity is standardized so that the mice receive a lethal dose of the organism (the lethal dose in the minimum inoculum of organism required to consistently kill 100 percent of the infected, non-treated control mice). The test compound in combination with the antibiotic is administered at various dosage levels, *p.o.* or *i.p.,* to groups of infected mice. At the end of the test, the activity of the mixture is assessed by counting the number of survivors among treated animals at a given dose. Activity is expressed as the percentage of animals which survive at a given dose, or calculated as a $PD_{50}$ (dose which protects 50% of the animals from infection). Compounds of the formulae (III) and (IV) are tested for *in vivo* activity in like manner, except that they are generally dosed alone, not in combination with other beta-lactam antibiotics.

In determining whether a particular strain of bacteria is sensitive to a particular compound of the formula (III) or (IV) it is not necessary to carry out an *in vivo* test. Instead, the MIC of a 1:1 mixture of a compound of the formula (I) or (II), wherein $R^1$ is hydrogen, and ampicillin or amoxicillin, as appropriate, are measured according to methods described above.

The ability of the compounds of formulae (I) and (II) to enhance the effectiveness of a beta-lactam antibiotic against beta-lactamase producing bacteria makes them valuable for coadministration with beta-lactam antibiotics in the treatment of bacterial infections in mammals, particularly man. In the treatment of a bacterial infection, the compound of the formula (I) or (II) can be comingled with the beta-lactam antibiotic, and the two agents thereby administered simultaneously. Alternatively, the compound of the

formula (I) or (II) can be administered as a separate agent during a course of treatment with a beta-lactam antibiotic. In some instances it will be advantageous to pre-dose the subject with the compound of the formula (I) or (II) before initiating treatment with a beta-lactam antibiotic.

When using a compound of formula (I) or (II) to enhance the effectiveness of beta-lactam antibiotic, a mixture of (I) or (II) with the beta-lactam antibiotic is administered preferably in formulation with standard pharmaceutical carriers or diluents. A pharmaceutical composition comprising a pharmaceutically acceptable carrier, a beta-lactam antibiotic and a compound of formula (I) or (II) will normally contain from about 5 to about 80 percent of the pharmaceutically acceptable carrier by weight.

When using the compounds of formula (I) or (II) in combination with another beta-lactam antibiotic, said compounds can be administered orally or parenterally, i.e. intramuscularly, subcutaneously or intraperitoneally. Although the prescribing physician will ultimately decide the dosage to be used in a human subject, the ratio of the daily dosages of the compounds of formula (I) or (II) and the beta-lactam antibiotic will normally be in the range of from about 1:3 to 3:1 by weight. Additionally, when using the compounds of formula (I) or (II) in combination with another beta-lactam antibiotic, the daily oral dosage of each component will normally be in the range of from about 10 to about 200 mg per kilogram of body weight and the daily parenteral dosage of each component will normally be about 10 to about 40 mg per kilogram of body weight. These daily doses will usually be divided. In some instances, the prescribing physician will determine that dosages outside these limits are necessary.

As will be appreciated by one skilled in the art, some beta-lactam compounds are effective when administered orally or parenterally, while others are effective only when administered by the parenteral route. When a compound of formula (I) or (II) is to be used simultaneously (i.e. comingled) with a beta-lactam antibiotic which is effective only on parenteral administration, a combination formulation suitable for parenteral use will be required. When a compound of formula (I) or (II) is to be used simultaneously (comingled) with a beta-lactam antibiotic which is effective orally or parenterally, combinations suitable for either oral or parenteral administration can be prepared. Additionally, it is possible to administer preparations of the compounds of formula (I) orally, while at the same time administering a further beta-lactam antibiotic parenterally; and it is also possible to administer preparations of the compounds of formula (I) parenterally, while at the same time administering the further beta-lactam antibiotic orally.

It is the capacity of compounds of the formula (III) or (IV) to hydrolyze and provide both the compounds of the formula (I) or (II), wherein $R^1$ is hydrogen, and ampicillin or amoxicillin, which enhances the activity and broadens the antibacterial spectrum of these compounds relative to the use of an equivalent amount of ampicillin or amoxicillin alone, particularly by the oral route.

When using the present antibacterial compounds of the formula (III) or (IV) for control of bacterial infections in a mammal, particularly man, the compound is administered alone, or mixed with pharmaceutically acceptable carriers or diluents. Said carrier or diluent is chosen on the basis of the intended mode of administration. For oral administration, tablets, capsules, lozenges, troches, powders, syrups, elixirs, aqueous solutions and suspensions, and the like are used, in accordance with standard pharamaceutical practice. The proportional ratio of active ingredient to carrier will naturally depend on the chemical nature, solubility and stability of the active ingredient, as well as the dosage contemplated. In the case of tablets for oral use, carriers which are commonly used include lactose, sodium citrate and salts of phosophoric acid. Various disintegrants such as starch, and lubricating agents, such as magnesium stearate, sodium lauryl sulfate and talc, are commonly used in tablets. For oral aministration in capsule form, useful diluents are lactose and high molecular weight polyethylene glycols, e.g. polyethylene glycols having molecular weights of from 2000 to 4000. When aqueous suspensions are required for oral use, the active ingredient is combined with emulsifying or suspending agents. If desired, certain sweetening and/or flavouring agents can be added. For parenteral administration, which includes intramuscular, intraperitoneal, subcutaneous, and intravenous use, sterile solutions of the active ingredient are usually prepared, and the pH of the solutions are suitably adjusted and buffered. For intravenous use, the total concentration of solutes should be controlled to render the preparation isotonic.

When using compounds of the formula (III) or (IV) to control bacterial infections, the daily dosage will be similar to that of other clinically used beta-lactam antibiotics. Although the prescribing physician will ultimately decide the dosage to be used in a human subject, the compounds of formula (III) or (IV) will normally be used orally at dosages in the range from about 20 to about 100 mg per kilogram of body weight per day, and parenterally at dosages from about 10 to about 100 mg per kilogram of body weight per day, usually in divided doses. In some instances, the prescribing physician will determine that dosage outside these limits are needed.

The present invention is illustrated by the following examples. However, it should be understood that the invention is not limited to the specific details of these examples. Unless otherwise specified, proton nuclear magnetic resonance spetra are 60 MHz.

## Example 1

Benzyl 6-alpha-bromo-6-beta-(benzyloxycarbonylaminomethyl)penicillanate and 6-beta-bromo-6-alpha-(benzyloxycarbonylaminomethyl)penicillanate

To a solution of benzyl 6,6-dibromopenicillanate (108.73 g, 0.242 mole) in 600 ml dry tetrahydrofuran (THF), cooled to −78°C, was added an ether solution of methyl magnesium bromide (83.5 ml of 2.9 M). After

stirring for 15 minutes at −78° a solution of benzyloxycarboxamidomethyl acetate (27 g, 0.121 mole) in 200 ml dry THF was added over 10 minutes. After stirring for an hour at −78° the reaction was quenched by the addition of 14.52 ml of acetic acid. The mixture was warmed to room temperature and volatiles removed *in vacuo* at less than 35°C. Ethyl acetate was added to dissolve the residue, and the solution washed with water (100 ml), aqueous $NaHCO_3$ (100 ml), and 2 × 100 ml water, then dried over $Na_2SO_4$ and concentrated *in vacuo* to 113 g of oily product. The oil was column chromatographed on 1.2 kg silica gel, eluting first with 6 liters of 1:1 hexane:chloroform and then with chloroform. The first 6 liters of eluate was discarded. Further eluate was collected in 25 ml fractions. Fraction numbers 181—190 were concentrated. The pnmr spectrum of the residue in $CDCl_3$ revealed benzyl 6-alpha-bromo-6-beta-(benzyloyxcarbonylaminomethyl)penicillanate: delta/TMS 1.37 (3H, s), 1.57 (3H, s), 3.86 (2H, d, j=6Hz), 4.42 (1H, s), 5.06 (2H, s), 5.12 (2H, s), 5.52 (1H, s) 7.25 (10H, s). Fraction numbers 201—249 were concentrated and the pnmr spectrum of this residue in $CDCl_3$ revealed benzyl 6-beta-bromo-6-alpha-(benzyloxycarbonylaminomethyl)-penicillanate: delta/TMS 1.36 (3H, s,), 1.60 (3H, s), 3.90 (2H, d, J=6.2 Hz), 4.47 (1H, s), 5.07 (2H, s), 5.14 (2H, s), 5.40 (1H, t, J=6.2), 5.47 (1H, s), 7.28 (5H, s), 7.30 (5H, s). The product from fraction numbers 171—240 was combined and concentrated to 22 g of foam and used in the experiment of Example 2.

## Example 2

Benzyl 6-beta-(Benzyloxycarbonylaminomethyl)penicillanate

To a solution of title products (epimeric mixture) of the preceding Example (22 g, 0.0413 mole) in 100 ml benzene was added tri-*n*-butyltin hydride (32.7 ml, 0.124 mole). The mixture was refluxed under $N_2$ for 2 hours, concentrated *in vacuo* to an oil and the oil triturated 4 × 100 ml hexane. The residual viscous oil was taken up in 70 ml of ether, from which title product crystallized over 1 hour [8.1 g in two crops] pnmr/$CDCl_3$/delta/TMS: 1.37 (3H, s), 1.57 (3H, s), 3.58 (3H, m), 4.34 (1H, s), 5.04 (2H, s), 5.12 (2H, s), 5.33 (1H, d, J=4Hz), 7.32 (10H, s).

## Example 3

Benzyl 6-beta-(Benzyloxycarbonylaminomethyl)-penicillanate 1-alpha-Oxide and
Benzyl 6-beta-)Benzyloxycarbonylaminomethyl)-penicillanate 1-beta-Oxide

To a solution of title product of the preceding Example (4.54 g, 0.01 mole) in 70 ml of ethyl acetate was added *m*-chloroperbenzoic acid (2.02 g, 0.01 mole) in 30 ml ethyl acetate. The mixture was stirred for 30 minutes at room temperature, washed 1 × 50 ml saturated $NaHCO_3$ and 2 × 50 ml $H_2O$, dried over $Na_2SO_4$ and concentrated *in vacuo* to a viscous oil. The oil was dissolved in 50 ml of ether and 10 ml of $CHCl_3$ and crystallization of title alpha-oxide induced by scratching [2.2 g, mp 123—124°C, pnmr/$CDCl_3$/delta/TMS 1.22 (3H, s), 1.51 (3H, s), 3.7 (3H, m), 4.34 (1H, s), 4.63 (1H, d, J=4Hz), 5.13 (2H, s), 5.22 (2H, s), 5.50 (1H, m), 7.34 (5H, s), 7.40 (5H, s)]. Concentration of mother liquor to dryness *in vacuo* gave the title beta-oxide as a viscous oil [2.5 g; pnmr/$CDCl_3$/delta/TMS 1.05 (3H, s), 1.60 (3H, s), 3.8 (3H, m), 4.63 (1H, s), 4.73 (1H, d, J=Hz), 5.13 (2H, s), 5.23 (2H, q), 5.70 (1H, m), 7.35 (5H, s), 7.39 (5H, s)].

## Example 4

Benzyl 6-alpha-(Benzyloxycarbonylaminomethyl)penicillanate 1-beta-Oxide

To title beta-oxide of the preceding Example (2.3 g, 4.9 mmoles) in 100 ml $CHCl_3$ was added 1,5-diazabicyclo[4.3.0]non-5-ene (DBN, 0.607 g, 4.9 mmoles). The mixture was stirred at room temperature for 15 minutes, diluted with 50 ml 1N HCl, and the layers separated. The organic layer was washed 2 × 50 ml $H_2O$, dried over $Na_2SO_4$ and concentrated *in vacuo* to an oil (2.3 g). The oil was column chromatographed on 100 g silica gel, eluting with 4:1 $CHCl_3$:ethyl acetate in 20 ml fractions. Fractions 41—70 were combined and concentrated *in vacuo* to yield title product as a viscous oil [0.9 g; pnmr/$CDCl_3$/TMS 1.03 (3H, s), 1.60 (3H, s), 3.67 (3H, m), 4.46 (1H, s), 4.88 (1H, m), 5.08 (2H, s), 5.17 (2H, q), 5.39 (1H, m), 7.32 (5H, s), 7.37 (5H, s)].

## Example 5

Benzyl 6-beta-(Benzyloxycarbonylaminomethyl)penicillanate 1,1-Dioxide

To a solution of title product of Example 2 (8.0 g, 0.0176 mole) in 200 ml ethyl acetate cooled to 0—5°C was added *m*-chloroperbenzoic acid (10.68 g, 0.0528 mole). The mixture was warmed to room temperature, stirred for 6 hours, recooled to 0—5°C and diluted with 50 ml of saturated $NaHSO_3$. The organic layer was separated, washed 2 × 50 ml saturated $NaHCO_3$ and 2 × 50 ml $H_2O$, dried over $Na_2SO_4$ and concentrated *in vacuo* to a viscous oil (8.6 g). The oil was chromatographed on 250 g silica gel, eluting with 19:1 $CHCl_3$:ethyl acetate in 25 ml fractions. Fractions 44—150 were combined and concentrated *in vacuo* to yield title product as a white gummy foam [7.6 g; pnmr/$CDCl_3$/delta/TMS 1.25 (3H, s), 1.49 (3H, s), 3.98 (3H, m), 4.45 (1H, s), 4.59 (1H, d, J = 4Hz), 5.09 (2H, s), 5.19 (2H, q), 5.36 (1H, br), 7.36 (10H, s)].

## Example 6

Benzyl 6-alpha-(Benzyloxycarbonylaminomethyl)penicillanate 1,1-Dioxide

By the procedure of Example 4, the title 1,1-dioxide of the preceding Example (3.3 g, 6.79 mmoles) was converted to present title product (3.1 g crude) and purified by column chromatography on 150 g silica gel, eluting with 1:9 ethyl acetate:$CHCl_3$ in 20 ml fractions. Fractions 26—37 were combined and concentrated *in vacuo* to yield purified title product, as a viscous oil which crystallized on standing [1.9 g; mp

112—113°C; pnmr/$CDCl_3$/delta/TMS 1.20 (3H, s), 1.49 (3H, s), 3.65 (3H, m), 4.32 (1H, s), 4.59 (1H, m), 5.07 (2H, s), 5.14 (2H, q), 5.30 (1H, br), 7.32 (10H, s)].

Present title product was also obtained by the further oxidation of the title product of Example 4 with excess *m*-chloroperbenzoic acid according to the method of Example 5.

Example 7

6-beta-(Aminoethyl)penicillanic Acid 1,1-Dioxide

Title product of Example 5 (1.9 g), THF (40 ml), $H_2O$ (40 ml) and 10% Pd/C (1.9 g) were combined and hydrogenated at 50 psig for 1 hour. Catalyst was recovered by filtration and THF removed from the filtrate *in vacuo*. The aqueous layer was washed with 30 ml ethyl acetate, freeze dried to a white powder and a first crystalline crop (0.26 g) obtained by trituration of the powder with 5 ml water. A second crop (0.14 g) crystallized on addition of 10 ml of acetone to the mother liquor and a third crop (0.35 g) by evaporating the second mother liquor to 2 ml and adding 50 ml of acetone. Total yield of title product was 0.75 g [pnmr/250 MHz/$D_2O$/delta/DSS 1.47 (3H, s), 1.59 (3H, s), 3.74 (2H, m), 4.36 (1H, td, J = 4, 5.5Hz), 4.45 (1H, s), 5.17 (1H, d, J = 4Hz)].

Example 8

6-alpha-(Aminomethyl)penicillanic Acid 1,1-Dioxide

By the method of the preceding Example, title product of Example 6 (1.7 g) was converted to present title product, except that crystalline product was obtained directly by concentration *in vacuo* following the ethyl acetate extraction [0.7 g; pnmr/250 MHz/$D_2O$/DSS 1.44 (3H, s), 1.59 (3H, s), 3.63 (2H, d, J = 5.5Hz), 4.07 (1H, td, J = 2, 5.5Hz), 4.31 (1H, s), 5.06 (1H, d, J = 2)].

Example 9

Benzyl 6-beta-Bromo-6-alpha-trifluoromethanesulfonyloxymethylpenicillanate

To a solution of trifluoromethanesulfonic anhydride (3.15 ml) in methylene chloride (20 ml) at room temperature was added a solution of benzyl 6-beta-bromo-6-alpha-(hydroxymethyl)penicillanate (6.232 g, 15.6 mmoles) and pyridine (1.89 ml) in methylene chloride (20 ml) and the mixture stirred and cooled in an ice bath for 45 minutes. The methylene chloride was evaporated under reduced pressure and the residue partitioned between ethyl acetate and water. The ethyl acetate phase was separated and the aqueous phase extracted with additional ethyl acetate. The combined ethyl acetate solutions were washed first with sodium bicarbonate solution at pH 8.3 and then with brine. After drying over anhydrous sodium sulfate, the solution was evaporated under reduced pressure to give title product as an orange solid [8.296 g; pnmr/$CDCl_3$/delta/TMS 1.41 (s, 3H), 1.63 (s, 3H), 4.51 (s, 1H), 4.87 (s, 2H), 5.14 (s, 2H), 5.44 (s, 1H), 7.30 (s, 5H)].

Example 10

Benzyl 6-alpha-Azidomethyl-6-beta-bromopenicillanate

Tetramethylguanidinium azide (2.96 g, 18.7 mmoles) was added to a solution of benzyl 6-beta-bromo-6-alpha-trifluoromethylsulfonyloxymethylpenicillanate (8.296 g, 15.6 mmoles) in chloroform (50 ml) at 10°C. The reaction mixture was stirred for one hour and then reduced to one third volume and filtered through a pad of silica gel. The pad was eluted with 10% ethyl acetate/chloroform (100 ml) and the eluate evaporated to give an amber oil [6.744 g; pnmr/$CDCl_3$/delta/TMS 1.38 (s, 3H), 1.61 (s, 3H), 3.96 (s, 2H), 4.53 (s, 1H), 5.17 (s, 2H), 5.40 (s, 1H), 7.34 (s, 5H)].

Example 11

Benzyl 6-alpha-Bromo-6-beta-trifluoromethanesulfonyloxymethylpenicillanate

Following the procedure of Example 9, benzyl 6-alpha-bromo-6-beta-hydroxymethylpenicillanate (0.548 g, 1.4 mmoles) in methylene chloride (4 ml) containing pyridine (0.17 ml) was reacted with a solution of trifluoromethanesulfonic anhydride (0.42 ml) in methylene chloride (3 ml) to give title product as an amber oil [641 mg; pnmr/$CDCl_3$/delta/TMS 1.43 (s, 3H), 1.62 (s, 3H), 4.52 (s, 1H), 4.88 (q, 2H), 5.19 (s, 2H), 5.62 (s, 1H), 7.35 (s, 5H)].

Example 12

Benzyl 6-alpha-Bromo-6-beta-azidomethylpenicillanate

To a solution of benzyl 6-alpha-bromo-6-beta-trifluoromethanesulfonyloxymethylpenicillanate (641 mg, 1.2 mmoles) in chloroform (10 ml) was added tetramethylguanadinium azide (229 mg, 1.2 mmoles) at 10°C. The reaction mixture was stirred for one hour and then evaporated under reduced pressure. The oily residue was filtered through a pad of silica gel and eluted therefrom with 10% ethyl acetate/chloroform. Evaporation of the eluate gave title product as an amber oil [420 mg; pnmr/$CDCl_3$/delta/TMS 1.43 (s, 3H), 1.61 (s, 3H), 3.91 (s, 2H), 4.48 (s, 1H), 5.15 (s, 2H), 5.57 (s, 1H), 7.37 (s, 5H)].

Example 13

Benzyl 6-alpha-(Aminomethyl)-6-beta-bromopenicillanate

Hydrogen sulfide was bubbled into a rapidly stirred solution of benzyl 6-alpha-azidomethyl-6-beta-bromopenicillanate (541 mg, 1.3 mmoles) and triethylamine (0.71 ml, 4 equivalents) in chloroform (10 ml)

for one hour. The reaction mixture was then evaporated *in vacuo* to a red oil. NMR data showed the residue to comprise the desired product contaminated with triethylamine [pnmr/$CDCl_3$/delta/TMS 1.39 (s, 3H), 1.64 (s, 3H), 3.35 (s, 2H), 4.51 (s, 1H), 5.16 (s, 2H), 5.35 (s, 1H), 7.33 (s, 5H)].

Example 14

Benzyl 6-beta-Bromo-6-alpha-benzyloxycarbonylaminomethyl)penicillanate

A solution of pyridine (0.14 ml) and benzyl 6-alpha-aminomethyl-6-beta-bromopenicillanate (239 mg, 0.6 mmoles) in methylene chloride (5 ml) was added via s syringe over a 5 minute period to a solution of benzylchloroformate in methylene chloride (5 ml) and the reaction mixture stirred in an ice bath under a nitrogen atmosphere for 75 minutes. The reaction mixture was evaporated *in vacuo* and the residue taken up in ethyl acetate/water. The pH was adjusted to 2.9 with dilute hydrochloric acid, the ethyl acetate phase separated and extracted with dilute sodium bicarbonate solution (pH 8.1), washed with brine and dried over anhydrous sodium sulfate. Evaporation under reduced pressure gave 312 mg which was taken up in chloroform and chromatographed on silica gel (15 g, 14 × 20 cm column) and eluted therefrom with 5% ethyl acetate/chloroform. Fractions of 4 ml each were collected. Fractions 14—27 were combined and evaporated under reduced pressure to give title product [168 mg, pnmr/$CDCl_3$/delta/TMS consistent with title product and identical with that of the same compound prepared in Example 1.

Example 15

Benzyl 6-beta-(Benzyloxycarbonylaminomethyl)penicillanate

A solution of tri(n-butyl)tin hydride (0.25 ml) and benzyl 6-beta-bromo-6-alpha-(benzyloxycarbonylaminomethyl)penicillanate (168 mg, 0.31 mmoles) benzene (4 ml) was refluxed for 2 hours. The benzene was then evaporated *in vacuo* and the residue triturated with hexane (3 × 2 ml). The remaining residue was then taken up in ethyl acetate/water, the ethyl acetate phase separated, washed with brine and dried over anhydrous sodium sulfate. Evaporation *in vacuo* gave 101 mg of an oil which was chromatographed on silica gel (4 g, 1 × 11 cm column), set up with chloroform and eluted with 5% ethyl acetate/chloroform. Fractions of 4 ml volume were collected. Fractions 3—5 were combined and evaporated to give title product (66 mg; identified by pnmr as identical with the product of Example 2).

Example 16

6-beta-(Benzylaminomethyl)penicillanic Acid 1,1-Dioxide

To a hazy solution of the beta-aminomethyl title product of Example 7 (0.3 g, 1.145 mmoles) in 30 ml methanol was added benzaldehyde (0.117 ml, 1.145 mmoles) followed by sodium cyanoborohydride (47.6 mg, 0.758 mmole). The mixture was stirred under $N_2$ for 30 minutes at room temperature. The reaction mixture was clarified by filtration and concentrated to a foam *in vacuo*. The foam was dissolved in 30 ml $H_2O$, extracted 2 × 20 ml ethyl acetate and freeze dried to title product as a white glass (130 mg; pnmr/$D_2O$/delta/DSS 1.57 (3H, s), 1.69 (3H, s), 3.7—4.4 (5H, m), 4.38 (1H, s), 5.21 (1H, d, J = 4), 7.56 (5H, s).

Example 17

6-beta-(2-Phenylethylaminomethyl)penicillanic Acid 1,1-Dioxide

Title beta-aminomethyl product of Example 7 (0.1 g) was reacted with phenylacetaldehyde (0.098 ml) by the method of the preceding Example. The reaction mixture was concentrated *in vacuo* and solids recovered by trituration with 20 ml ethyl acetate. The solids were dissolved in water, the solution combined with a water extract of the ethyl acetate phase and freeze dried to yield title product [40 mg, pnmr/$D_2O$/delta/DSS 1.56 (3H, s), 1.70 (3H, s), 3.0—4.0 (6H, m), 4.28 (1H, m), 4.41 (1H, s), 5.24 (1H, d, J = 4), 7.48 (5H, s)].

Example 18

6-beta-(4-Picolylaminomethyl)penicillanic Acid 1,1-Dioxide

Title beta-aminomethyl product of Example 7 (0.1 g, 0.38 mmole) was slurried in 4 ml of water. 4-Pyridinecarbaldehyde (0.040 ml, 0.42 mmole) and then sodium cyanoborohydride (15.8 mg, 0.25 mmole) were added and the mixture stirred under nitrogen for 30 minutes at room temperature. The hazy solution was clarified, concentrated *in vacuo*, the residue taken up in 5 ml $H_2O$, extracted 10 ml ethyl acetate and freeze dried to yield title product as a white glass [0.1 g; pnmr/$D_2O$/delta/DSS 1.53 (3H, s), 1.64 (3H, s), 3.3—4.1 (5H, m), 4.35 (1H, s), 5.14 (1H, d, J = 4Hz), 8.1 (4H, m)].

Example 19

6-beta-(3-Picolylaminomethyl)penicillanic Acid 1,1-Dioxide

The procedure of the preceding Example, substituting 3-pyridinecarbaldehyde (0.039 ml) for 4-pyridinecarbaldehyde, gave present title product [70 mg, pnmr/$D_2O$/delta/DSS 1.59 (3H, s), 1.71 (3H, s), 3.7—4.5 (5H, m), 4.45 (1H, s), 5.23 (1H, d, J = 4Hz), 8.1 (4H, m)].

Example 20

6-alpha-(Benzylaminomethyl)penicillanic Acid 1,1-Dioxide

The title alpha-aminomethyl compound of Example 8 (0.5 g, 1.91 mmoles) was reacted with benzaldehyde (0.194 ml, 1.91 mmole) and sodium cyanoborohydride (79.4 mg, 1.259 g) in a total of 26 ml of

methanol according to the method of Example 16. Following the 30 minutes stir period the reaction mixture was clarified by filtration and concentrated *in vacuo* to a foamy residue. The residue was dissolved in 50 ml of ethyl acetate and crude product (0.45 g) precipitated by adding hexane. Crude product (0.35 g) was dissolved in 30 ml of water, extracted 2 × 30 ml ethyl acetate, and concentrated *in vacuo* to yield title product as a glass [0.28 g; pnmr/$D_2O$/delta/DSS 1.54 (3H, s), 1.67 (3H, s), 3.47 (2H, m), 4.03 (3H, m), 4.33 (1H, s), 4.98 (1H, d, J = 2), 7.53 (5H, s)].

Example 21

6-alpha-(2-Phenylethylaminomethyl)penicillanic Acid 1,1-Dioxide

The procedure of the preceding Example, substituting phenylacetaldehyde (0.446 ml) for benzaldehyde provided title product. Isolation was modified in that the crude product initially precipitated as a gum when the hexane was added to the ethyl acetate. The gum was isolated by decantation and partitioned between 20 ml ethyl acetate and 20 ml $H_2O$ and insoluble material (100 mg) removed by filtration. The aqueous layer was freeze dried to yield purified title product as a yellow solid [0.18 g, pnmr/$D_2O$/delta/DSS 1.55 (3H, s), 1.70 (3H, s), 2.9—4.0 (7H, m), 4.34 (1H, s), 5.10 (1H, d), 7.43 (5H, s)].

Example 22

6-alpha-(4-Picolylaminomethyl)penicillanic Acid 1,1-Dioxide

The procedure of Example 20, substituting 4-pyridinecarbaldehyde (0.1892 ml) for benzaldehyde gave title product. Isolation was modified in that, following filtration of the reaction mixture, the filtrate was concentrated *in vacuo* to a yellow foam, which was triturated with ethyl acetate, taken up in 10 ml $H_2O$ and washed with 20 ml fresh ethyl acetate. The aqueous layer was reconcentrated *in vacuo* to yield title product as a second yellow foam (0.38 g; pnmr/$D_2O$/delta/DSS 1.57 (3H, s), 1.70 (3H, s), 3.39 (2H, m), 4.0 (3H, m), 4.32 (1H, s), 5.01 (1H, d, J = 2), 8.1 (4H, m)].

Example 23

6-alpha-(3-Picolylaminomethyl)penicillanic Acid 1,1-Dioxide

The procedure of the preceding Example substituting 3-pyridinecarbaldehyde (0.182 ml) for 4-pyridinecarbaldehyde gave title product. Isolation was modified in that the initially isolated yellow foam was taken up in 10 ml $H_2O$, extracted 2 × 10 ml ethyl acetate, and the aqueous layer freeze dried to yield title product as a second yellow foam [0.39 g, pnmr/$D_2O$/delta/DSS 1.57 (3H, s), 1.70 (3H, s), 3.43 (2H, m), 4.1 (3H, m), 4.30 (1H, s), 5.00 (1H, d, J = 2), 8.1 (4H, m).

Example 24

6-alpha-(4-Hydroxybenzylaminomethyl)penicillanic Acid 1,1-Dioxide

By the procedure of the preceding Example, title product of Example 8 (0.1 g, 0.38 mmole)] was reacted with 4-hydroxybenzaldehyde (46.6 mg, 0.38 ml) and sodium cyanoborohydride (15.8 mg, 0.25 mmole) in 5 ml methanol to produce freeze dried title product as a white solid [0.1 g, pnmr/$D_2O$/delta/DDS 1.53 (3H, s), 1.68 (3H, s), 3.52 (2H, m), 4.1 (3H, m), 4.33 (1H, s), 5.00 (1H, d, J = 2), 7.1 (4H, m).

Example 25

Benzyl 6-alpha-(Benzyloxycarbonylaminomethyl)penicillanate and Benzyl 6-beta-(Benzyloxycarbonylaminomethyl)penicillanate

The required Grignard agent was prepared essentially according to the method of DiNinno *et al.,* J. Org. Chem. 42, pp. 2960—2965 (1977). Thus benzyl 6-alpha-iodopenicillanate was dissolved in 75 ml of dry tetrahydrofuran and cooled to −78°C under dry $N_2$. Methylmagnesium bromide (5.6 ml of 3M in ether) was added dropwise. After stirring an additional 15 minutes, a solution of benzyloxycarbonylaminomethyl acetate (1.87 g) in 25 ml of dry tetrahydrofuran was added in one portion. After a second 15 minutes of stirring at −78°C, acetic acid (2 ml) was added, the mixture warmed to 0°C and evaporated *in vacuo*. The residue was distributed between 250 ml ethyl acetate and 50 ml of water. The organic layer was separated, washed 1 × 100 ml saturated $NaHCO_3$ and 2 × 100 ml brine, dried over $Na_2SO_4$, and evaporated *in vacuo* to an oil (7.3 g). The oil was chromatographed on 250 g silica gel, eluting with 1:10 ethyl acetate:chloroform in 20 ml fractions. Fractions 20—24 contained 1.3 g of a side product (oil); fractions 25—34 contained 0.62 g of a 3:2 beta:alpha mixture of title products by pnmr assay. Fractions 35—60 contained 2.2 g of a 3:1 alpha:beta mixture of title products.

Example 26

Benzyl 6-alpha-Bromo-6-beta-(1R-trifluoromethanesulfonyloxyethyl)penicillanate

Benzyl 6-alpha-bromo-6-beta-(1R-hydroxyethyl)penicillanate (DiNinno *et al.,* J. Org. Chem. 42, pp. 2960—2965, 1977; 20.28 g, 0.0489 mole) was dissolved in 400 ml $CH_2Cl_2$ and chilled with an acetone-ice bath. Pyridine (7.91 ml, 2 equivalents) was added and then, dropwise, trifluoromethanesulfonic anhydride (11.58 ml, 1.4 equivalents) maintaining temperature ±5°. The mixture was stirred at 0° for 30 minutes, diluted with $CH_2Cl_2$, washed sequentially with saturated $NaHCO_3$, $H_2O$ and saturated HaCl, evaporated *in vacuo*, and diluted with 1:1 hexane, thereby crystallizing title product, 18.71 g; pnmr/$CDCl_3$/delta/TMS: 1.42 (3H, s), 1.55 (3H, d, J = 6Hz), 1.66 (3H, s), 4.58 (1H, s), 5.20 (2H, s), 5.34 (1H, q, J = 6Hz), 5.56 (1H, s), 7.38 (5H, s).

Example 27

Benzyl 6-alpha-Brom-6-beta-(1S-azidoethyl)penicillanate

Title product of the preceding Example (34.4 g, 0.0628 mole) was dissolved in 400 ml of $CH_2Cl_2$. Tetrabutylammonium azide (25.0 g, 1.4 equivalents) in 100 ml $CH_2Cl_2$ was added dropwise and the mixture stirred for one hour at 25°C. The $CH_2Cl_2$ was stripped *in vacuo* and the residual oil filtered through 500 g of silica gel on a sintered glass funnel, eluting with 2 liters $CH_2Cl_2$. Evaporation gave title product as a pale yellow oil, 27 g; pnmr/$CDCl_3$/delta/TMS: 1.40 (3H, s), 1,53 (3H, d, J = 6.3Hz), 1.57 (3H, s), 4.10 (1H, q, J = 6.3Hz), 4.47 (1H, s), 5.19 (2H, s), 5.61 (1H, s), 7.34 (5H, s).

Example 28

Benzyl 6-beta-(1S-Aminoethyl)-6-alpha-bromopenicillanate

Title product of the preceding Example (27.0 g, 0.0613 mole) was dissolved in 300 ml $CHCl_3$. Triethylamine (35 ml, 4 equivalents) was added and then $H_2S$ was bubbled through the reaction mixture for 3.5 hours. The reaction mixture was then flushed with $N_2$ for 0.5 hour and stripped of $CHCl_3$ *in vacuo*. The resulting oil was distributed between ether and IN HCl (200 ml). The ether layer was extracted 3 × 200 ml fresh 1N HCl. The combined aqueous layers were diluted with ethyl acetate and the pH of the two phase system adjusted to 8.5. The organic layer was separated and evaporated to yield title product as an oil (6.34 g). The original ether layer was diluted with water and the pH adjusted to 8.5. The ether layer was separated and evaporated to yield additional title product, 6.0 g, pnmr/$CDCl_3$/delta/TMS: 1.28 (3H, d, J = 6.3Hz), 1.40 (3H, s), 1.58 (3H, s), 3.34 (1H, q, J = 6.3Hz), 4.45 (1H, s), 5.16 (2H, s), 5.54 (1H, s), 7.33 (5H, s).

Example 29

Benzyl 6-beta-(1S-Benzyloxycarbonylaminoethyl)-6-alpha-bromo-penicillanate

Benzyl chloroformate (5.10 ml, 1.2 equivalents) was dissolved in 20 ml of $CH_2Cl_2$, chilled in an acetone-ice bath. A solution of title product of the preceding Example (12.34 g, 0.02980 mole) in 50 ml of $CH_2Cl_2$ containing diisopropylethylamine (7.78 ml, 1.5 equivalents) was added dropwise, maintaining the temperature 0—5°C with an ice water bath. The reaction mixture was stirred for 20 minutes, stripped of $CH_2Cl_2$ *in vacuo,* diluted with ethyl acetate and water (pH was noted to be 8.2), and adjusted to pH 2.0 with dilute HCl. The organic layer was separated, washed with $H_2O$ and then saturated NaCl and evaporated to yield title product as a pale yellow oil, 17 g; pnmr/$CDCl_3$/delta/TMS: 1.35 (3H, s), 1.38 (3H, d), 1.55 (3H, s), 4.26 (1H, m), 4.45 (1H s), 4.97 (1H, d), 5.08 (2H, S), 5.14 (2H, s), 5.53 (1H, s), 7.33 (10H, s).

Example 30

Benzyl 6-beta-(1S-Benzyloxycarbonylaminoethyl)penicillanate

Title product of the preceding Example (16.33 g, 0.0298 mole) was dissolved in 200 ml benzene. Tri-*n*-butyltin hydride (23.5 ml, 3 equivalents) was added and the mixture refluxed 3 hours. Benzene was removed *in vacuo* and title product crystallized from the residue by addition of hexane, 9.26 g; pnmr/$CDCl_3$/delta/TMS: 1.39 (3H, d, J = 6Hz), 1.40 (3H, s), 1.63 (3H, s), 3.62 (1H, dd, J = 4, 11Hz), 4.12 (1H, m), 4.39 (1H s), 4.81 (1H, d, J = 8Hz), 5.08 (2H s), 5.17 (2H, s), 5.32 (1H, d, J = 4Hz), 7.32 (5H, s), 7.36 (5H, s).

Example 31

Benzyl 6-beta-(1S-Benzyloxycarbonylaminoethyl)penicillanate 1,1-Dioxide

By the procedure of Example 5, title product of the preceding Example (4.0 g, 0.0085 mole) was converted to present title product, isolated as a white foam, 4.25 g; pnmr/$CDCl_3$/delta/TMS: 1.21 (3H, s), 1.33 (3H, d, J = 6Hz), 1.48 (3H, s), 4.20 (1H, m), 4.35 (1H, m), 4.40 (1H, s), 4.53 (1H, d, J = 4.5Hz), 5.08 (2H, s), 5.17 (2H, q), 5.45 (1H, d, J = 7Hz), 7.30 (5H, s), 7.35 (5H, s).

Example 32

6-beta-(1S-Aminoethyl)penicillanic Acid 1,1-Dioxide

Using 180 mg of 5% Pd/C as catalyst, title product of the preceding Example (175 mg) was converted to present title product by the procedure of Example 7. After recovery of the catalyst, the combined mother liquor and tetrahydrofuran/$H_2O$ washes were stripped of the former *in vacuo* and title product recovered by freeze drying the aqueous residue overnight, ir (KBr): 1765 $cm^{-1}$; pnmr/$D_2O$/delta/DDS: 1.47 (3H, s), 1.53 (3H, d), 1.60 (3H, s), 4.35 (2H, m), 4.37 (1H, s), 5.12 (1H, d, J = 4Hz).

Example 33

Benzyl 6-alpha-(1S-Benzyloxycarbonylaminoethyl)penicillanate 1,1-Dioxide

Title product of Example 31 (4.25 g, 0.0085 mole) was dissolved in 100 ml $CH_2Cl_2$. DBN (0.96 ml, 1 equivalent) was added dropwise and the mixture stirred three minutes at 25°, then quenched by adding to 1 ml (2 equivalents) of glacial $CH_3CO_2H$. The quenched mixture was diluted with $CH_2Cl_2$, washed in sequence with dilute HCl (pH 2.5), water and saturated NaCl, and evaporated *in vacuo* to yield crude title product as a white foam, 4.5 g. Column chromatography on silica gel, using 9:1 $CH_2Cl_2$:ethyl acetate as eluant, gave purified title product, white foam, 2.83 g, p-nmr/$CDCl_3$/delta/TMS: 1.21 (3H, s), 1.35 (3H, d, J = 7Hz), 1.51 (3H, s), 3.78 (1H, dd, J = 2, 4Hz), 4.27 (1H, m), 4.36 (1H, s), 4.56 (1H, d, J = 2Hz), 5.09 (2H, s), 5.16 (2H, q), 7.3 (10H, s).

Example 34

6-alpha-(1S-Aminoethyl)penicillanic Acid 1,1-Dioxide

5% Pd/C (4.5 g) was slurried in 50 ml $H_2O$ and prehydrogenated for 1.5 hours at 25°C/50 psig. Title product of the previous Example (2.83 g) was dissolved in 50 ml ethyl acetate, added to the prehydrogenated aqueous catalyst slurry, and hydrogenated at 25°/50 psig for 0.5 hour. Catalyst was recovered by filtration. The aqueous layer was separated. Title product crystallized in three crops (1.1 g total weight) as the aqueous layer was concentrated; pnmr/$D_2O$/delta/DDS: 1.47 (3H, s), 1.52 (3H, d), 1.62 (3H, s), 4.05 (2H, m), 4.28 (1H, s), 5.10 (1H, d); ir (KBr) 1787 $cm^{-1}$.

Example 35

Benzyl 6-beta-Bromo-6-alpha-(1-trifluoromethylsulfonyloxyethyl)penicillanate

By the procedure of Example 26, benzyl 6-beta-bromo-6-alpha-(1-hydroxymethyl)penicillanate (a mixture of side chain epimers; DiNinno *et al., loc cit.;* 8.90 g, 0.0214 mole) was converted to present title products. On addition of 1:1 hexane:ether a first crop of solids crystallized (4.70 g) which was primarily the 1R side chain epimer. (If desired, this epimer is carried through the procedures of Examples 25—30 to yield the title product of Example 32). The second crop of solids (2.40 g) and the third crop (an oil, 1.60 g) were mixed side chain epimers of title product, primarily the 1S side chain epimer; pnmr/$CDCl_3$/delta/TMS: 1.41 (3H, s), 1.65 (3H, s), 1.78 (3H, d, J = 6.5Hz), 4.56 (1H, s), 5.22 (2H, s), 5.37 (1H, q, J = 6.5Hz), 5.48 (1H, s), 7.4 (5H, s).

Example 36

Benzyl 6-alpha-(1-Azidoethyl)-6-beta-bromopenicillanate

Second and third crops (primarily 1S side-chain epimer) of title product of the preceding Example (4.0 g, 0.0073 mole) were converted to present title product (primarily 1R side chain epimer) by the procedure of Example 27. This product was isolated as a pale yellow oil, 2.91 g, pnmr/$CDCl_3$/delta/TMS: 1.40 (3H, s), 1.56 (3H, d, J = 6.5Hz), 4.00 (1H, q, J = 6.5Hz), 4.48 (1H, s), 5.16 (2H, s), 5.34 (1H, s), 7.32 (5H, s).

Example 37

Benzyl 6-alpha-(1R-Aminoethyl)-6-beta-bromopenicillanate and Benzyl 6-alpha-(1S-Aminoethyl)-6-beta-bromopenicillanate

Title product of the previous Example (primarily the 1R side chain epimer; 2.91 g, 0.0066 mole) was converted to present title products according to the procedure of Example 28. Following removal of $CHCl_3$ used as reaction solvent, the residue was distributed between ether and 1N HCl (200 ml). The ether layer was separated and extracted 1 × 200 ml 1N HCl. The combined aqueous layers were layered with ethyl acetate and the pH adjusted to 8.5. The aqueous layer was separated and extracted with fresh ethyl acetate. The ethyl acetate layers were combined and evaporated to yield a mixture of title products (primarily the 1R isomer) as an oil, 1.53 g. The isomers were separated by column chromatography on 200 g silica gel, eluting with 1:1 $CH_2Cl_2$:ethyl acetate, monitoring by tlc (3:2 $CH_2Cl_2$:ethyl acetate). Clean, faster moving fractions ($R_f$ 0.52) were combined and evaporated to yield title 1S isomer (0.172 g), which, if desired, is converted by the procedures of Examples 29—32 to title product of Example 34. Middle cuts gave a mixture of title products (0.45 g), which, if desired, is rechromatographed to yield additional pure products. Clean, slower moving fractions ($R_f$ 0.47) were combined to yield title 1R side-chain epimer, 0.674 g, pnmr/$CDCl_3$/delta/TMS: 1.29 (3H, d, J = 6Hz), 1.38 (3H, s), 1.64 (3H, s), 3.29 (1H, q, J = 6Hz), 4.51 (1H, s), 5.17 (2H, s), 5.38 (1H, s), 7.33 (5H, s).

Example 38

Benzyl 6-beta-Bromo-6-alpha-(1R-benzyloxycarbonylaminoethyl)penicillanate

By the procedure of Example 29, 1R side chain title product of the preceding Example (0.674 g, 0.0016 mole) was converted to present title product, 0.877 g, $R_f$ 0.85 (1:1 $CH_2Cl_2$:ethyl acetate).

Example 39

Benzyl 6-beta-(1R-Benzyloxycarbonylaminoethyl)penicillanate

By the procedure of Example 30, title product of the preceding Example (0.877 g, 0.0016 mole) was converted to present title product. After stripping, the benzene used as solvent, the residue was triturated 4 × 50 ml with hexane to yield crude product as an oil (627 mg). The oil was chromatographed on silica gel eluting with 19:1 chloroform:ethyl acetate to yield purified title product, 569 mg, pnmr/$CDCl_3$/delta/TMS: 1.10 (3H, d, J = 6.5Hz), 1.36 (3H, s), 1.60 (3H, s), 3.41 (1H, dd, J = 4, 11Hz), 4.16 (1H, m), 4.43 (1H, s), 5.08 (2H, s), 5.13 (2H, s), 5.32 (1H, d, J = 4Hz), 7.29 (5H, s), 7.33 (5H, s).

Example 40

Benzyl 6-beta-(1R-Benzyloxycarbonylaminoethyl)penicillanate 1,1-Dioxide

By the procedure of Example 5, title product of the preceding Example (0.569 g, 0.0012 mole) was converted to present title product, 0.681 g, pnmr/$CDCl_3$/delta/TMS: 1.22 (3H, s), 1.27 (3H, d), 1.47 (3H, s), 3.94 (1H, dd, J = 4, 12Hz), 4.42 (1H, s), 4.48 (2H, m), 5.04 (2H, s), 5.13 (2H, q), 5.40 (1H, d, J = 8), 7.27 (5H, s), 7.32 (5H, s).

Example 41

6-beta-(1R-Aminoethyl)penicillanic Acid, 1,1-Dioxide

Using 400 mg of 5% Pd/C, title product of the preceding Example (202 mg) was converted to present title product, 50 mg; ir (KBr) 1788 $cm^{-1}$; pnmr/$D_2O$/delta/DDS: 1.45 (3H, s), 1.51 (3H, d), 1.57 (3H, s), 4.27 (1H, m), 4.33 (1H, s), 4.85 (1H, m), 5.15 (1H, d, J = 4Hz).

Example 42

Benzyl 6-alpha-(1R-Benzyloxycarbonylaminoethyl)penicillanate 1,1-Dioxide

By the procedure of Example 33, using 19:1 $CHCl_3$:ethyl acetate as eluant in the chromatography, title product of Example 40 (368 mg) was converted to present title product, 285 mg; pnmr/$CDCl_3$/delta/TMS: 1.23 (3H, s), 1.33 (3H, d, J = 6.5Hz), 1.50 (3H, s), 3.61 (1H, dd, J = 2, 9Hz), 4.28 (1H, m), 4.34 (1H, s), 4.67 (1H, d, J = 2Hz), 4.98 (1H, d), 5.07 (2H, s), 5.18 (2H, q), 7.30 (5H, s), 7.35 (5H, s).

Example 43

6-alpha-(1R-Aminoethyl)penicillanic Acid 1,1-Dioxide

By the procedure of Example 41, title product of the preceding Example (285 mg) was converted to present title product (132 mg; ir (KBr) 1768 $cm^{-1}$; pnmr/$D_2O$/delta/DDS: 1.47 (3H, s), 1.54 (3H, d), 1.61 (3H, s), 4.03 (2H, m), 4.44 (1H, s), 5.10 (1H, d, J = 2).

Example 44

6-alpha-(Benzyloxycarbonylaminomethyl)penicillanic Acid 1,1-Dioxide

*Method A*

Title product of Example 6 (11.2 g) in THF (70 ml) and $H_2O$ (50 ml) in the presence of 6 g 10% Pd/C was partially hydrogenated at 50 psig for 30 minutes. Catalyst was removed by filtration over a pad of diatomaceous earth, THF was distilled from the filtrate *in vacuo,* and the aqueous residue was extracted with 100 ml ethyl acetate. The organic layer was separated, dried ($Na_2SO_4$) and evaporated to yield title product as a foam, 3.0 g; pnmr/$CDCl_3$/TMS 1.40 (3H, s), 1.55 (3H, s), 3.70 (3H, m), 4.31 (1H, s), 4.58 (1H, m), 5.04 (2H, s), 7.24 (5H, s).

The aqueous layer was concentrated to yield crystalline 6-alpha-(aminomethyl)penicillanic acid 1,1-dioxide, 3.1 g, having pnmr fully identical with the same completely hydrogenated product of Example 8.

*Method B*

Title product of Example 8 (3.0 g, 11.45 moles) was dissolved in 100 ml 1:1 $H_2O$:methanol. The pH was adjusted and maintained at 8.3—8.7 as benzyl chloroformate (1.79 g, 12.59 mole) was added dropwise over several minutes. Following a brief period of stirring the pH was adjusted to 6.0 with 1N HCl and THF removed by distillation *in vacuo.* The aqueous residue was extracted with 30 ml of ethyl acetate and the extract discarded. Fresh ethyl acetate (50 ml) was added and the pH adjusted to 1.8 with 1N HCl. The aqueous layer was extracted with 50 ml fresh ethyl acetate. The combined organic layer and extract was washed 1 × 50 ml saturated NaCl, dried ($Na_2SO_4$) and evaporated *in vacuo* to yield title product as a foam, 3.7 g, having pnmr identical with that of title product obtained according to Method A immediately above.

Example 45

Pivaloyloxymethyl 6-alpha-(Benzyloxycarbonylaminomethyl)penicillanate 1,1-Dioxide

The title product of the preceding Example 6.75 g, 17 mmoles) and N,N-diisopropylethylamine (3.34 ml, 18.7 mmoles) were dissolved in dimethylformamide (50 ml), chloromethyl pivalate (2.72 ml, 18.7 mmoles) were added, and the mixture allowed to stir at ambient temperature for 20 hours. The reaction mixture was diluted with ethyl ether (300 ml), washed with water (2 × 100 ml), dried ($Na_2SO_4$) and evaporated *in vacuo* to an oil. The oil was dissolved in 100 ml ether, washed 3 × 50 ml $H_2O$, dried ($Na_2SO_4$) and concentrated *in vacuo* to yield purified title product as a viscous oil, 4.4 g, pnmr/$CDCl_3$/TMS 1.20 (9H, s), 1.34 (3H, s), 1.51 (3H, s), 3.64 (3H, m), 4.31 (1H, s), 4.60 (1H, d), 5.04 (2H, s), 5.71 (2H, q), 7.24 (5H, s).

Example 46

*p*--Toluenesulfonate Salt of Pivaloyloxymethyl 6-alpha-(Aminomethyl)penicillante 1,1-Dioxide

Title product of the preceding Example (1.8 g, 3.53 mmoles) was hydrogenated in a mixture of THF (40 ml) and $H_2O$ (20 ml) over 1.8 g of 10% Pd/C in the presence of pyridinium *p*-toluenesulfonate (1.77 g, 7.06 mmoles) at 50 psig for 1.5 hours. The catalyst was recovered by filtration over diatmoaceous earth and the filtrate stripped of THF *in vacuo,* during which the title product crystallized, 1.2 g, mp 214—215°C (dec.); pnmr/DMSO-$d_6$/TMS 1.16 (9H, s), 1.32 (3H, s), 1.48 (3H, s), 2.28 (3H, s), 3.34 (2H, m), 3.82 (1H, m), 4.60 (1H, s), 5.14 (1H, d, J = 2Hz), 5.75 (2H, ABq), 7.23 (4H, ABq).

Anal. Calcd. for $C_{15}H_{24}O_7N_2S.C_6H_7SO_3H$: C, 48.16; H, 5.88; N, 5.11.
Found: C, 48.31; H, 6.11; N, 5.08.

Example 47

Chloromethyl 6-[D-(2-azido-2-phenylacetamido)]penicillanate

A solution of 12.0 g (0.03 mole) 6-[D-(2-azido-2-phenylacetamido)penicillanic acid sodium salt, 25 ml water was combined with 100 ml methylene chloride and 10.17 g (0.03 mole) tetrabutylammonium hydrogen sulfate. The mixture (pH 3.0) was adjusted to pH 7.5 with sodium bicarbonate, the organic layer is separated and the aqueous layer was extracted with 2 × 100 ml methylene chloride. The combined organic layers were dried ($Na_2SO_4$) and the solvent evaporated to yield a solid residue. The residue was triturated with ethyl acetate (300 ml), filtered, the cake washed with ethyl acetate followed by ethyl ether and dried under nitrogen to afford 16.5 g (89%) of tetrabutylammonium salt.

A mixture of 12.32 g (0.02 mole) of the above salt was combined with 70 ml chloroiodomethane and the mixture stirred overnight at ambient temperature. The reaction mixture was concentrated to dryness and the residue purified by chromatography on 600 g silica gel, eluting with 1:1 ethyl acetate/hexane by volume to afford 8.1 g (95%) of the desired chloromethyl ester as a pale yellow viscous oil, pnmr/$CDCl_3$: 1.58 (s, 3H), 1.68 (s, 3H), 4.45 (s, 1H), 5.1 (s, 1H), 5.5—5.9 (dd m, 4H), 7.2 (d, 1H) and 7.4 (s, 5H) ppm.

Example 48

Iodomethyl 6-[D-(2-Azido-2-phenylacetamido)]penicillanate

Title product of the preceding Example (1.45 g, 0.00342 mole) in 30 ml acetone was purged 3 minutes with $N_2$. NaI (2.55 g, 0.01714 mole) was added and the resulting solution stirred 16 hours at ambient temperature. The reaction mixture was clarified by filtration, the filtrate concentrated *in vacuo,* and the residue taken into 75 ml $CHCl_3$ and filtered. The $CHCl_3$ filtrate was washed 2 × 30 ml saturated NaCl, dried ($Na_2SO_4$) and concentrated to yield title product as a foam, 1.23 g, pnmr/$CDCl_3$/TMS/delta (ppm): 1.53 (3H, s), 1.64 (3H, s), 4.37 (1H, s), 5.05 (1H, s), 5.56 (2H, m, J = 4, 11 Hz), 5.87 (2H, ABq), 7.31 (5H, s).

Example 49

6-alpha-(Benzyloxycarbonylaminomethyl)-1,1-dioxopenicillanoyloxymethyl 6-[D-(2-Azido-2-phenylacetamido)]penicillanate

Title product of Example 16 (0.56 g, 1.43 mmoles) was dissolved in 50 ml $CH_2Cl_2$. $H_2O$ (20 ml) was added and the pH adjusted to 8.6 with 1N NaOH. $NaHCO_3$ (0.121 g, 1.43 mmoles) was added followed by tetrabutylammonium hydrogen sulfate (0.488 g, 1.43 mmoles) in portions, while maintaining pH 8.0—8.3 with 1N NaOH, until near the end of the addition, when the pH was allowed to drop to 7.0. After stirring the mixture 15 minutes, the layers were separated. The aqueous layer was extracted 1 × 30 ml fresh $CH_2Cl_2$. The combined organic layer and extract was dried ($Na_2SO_4$) and concentrated *in vacuo* to yield tetrabutylammonium 6-alpha-(benzyloxycarbonylaminomethyl)penicillanate 1,1-dioxide as a foam. The latter was dissolved in 20 ml acetone and added to a solution of title product of the preceding Example (0.714 g, 1.43 mmoles) in 15 ml acetone and the mixture stirred 1 hour at ambient temperature, concentrated *in vacuo* and the residue slurried in 30 ml of ethyl acetate to yield crystalline tetrabutylammonium iodide (0.42 g). The filtrate was evaporated to a foam (1.2 g) which was chromatographed on 100 g silica gel eluting with 20% ethyl acetate/$CHCl_3$ in 20 ml fractions. Clean product fractions ($R_f$ 0.22 on tlc with the same eluant) were combined and concentrated *in vacuo* to yield purified title product as a foam, 0.61 g, pnmr/$CDCl_3$/delta (ppm): 1.33 (3H, s), 1.48 (3H, s), 1.52 (3H, s), 1.59 (3H, s), 3.65 (3H, m), 4.33 (1H, s), 4.42 (1H, s), 4.61 (1H, s [br]), 5.05 (3H, s), 5.58 (5H, m), 7.24 (5H, s), 7.32 (5H, s).

Example 50

Di(*p*-toluenesulfonate) Salt of 6-alpha-(Aminomethyl)-1,1-dioxopenicillanoyloxymethyl 6-[D-2-Amino-2-phenylacetamido]penicillanate

Pd/C (10%, 2 g) was prehydrogenated in 20 ml of water. A solution of title product of the preceding Example (0.96 g, 1.226 mmoles) in 30 ml THF and then pyridinium *p*-toluenesulfonate (0.615 g, 2.452 mmoles) were added, and the mixture hydrogenated for 1.5 hours at 50 psig. Catalyst was recovered by filtration over diatomaceous earth with THF and $H_2O$ wash. THF was removed from the combined filtrate and washes by concentration *in vacuo*. The aqueous residue was extracted 3 × 30 ml ethyl acetate and freeze dried to yield title product, 0.66 g, contaminated with unreduced benzyloxycarbonyl compound. Fresh 10% Pd/C (1.0 g) was prehydrogenated in 20 ml of $H_2O$. The contaminated title product (0.5 g) was dissolved in 30 ml THF and then added to the prereduced catalyst slurry. Finally, fresh pyridinium *p*-toluenesulfonate (0.315 g) was added and the mixture hydrogenated at 50 psig for 1.5 hours. Catalyst was recovered and purified title product recovered as above, 0.5 g, pnmr/DMSO-$d_6$/TMS/delta (250 MHz): 1.35 (6H, br. s), 1.47 (6H, s), 2.30 (6H, s), 3.38 (2H, m), 3.94 (1H, m, 4.45 (1H, s), 4.72 (1H, s), 5.08 (1H, br. s), 5.31 (1H, br. s), 5.45 (1H, d, J = 4Hz), 5.60 (1H, m), 5.93 (2H, m), 7.32 (8H, ABq), 7.48 (5H, m).

Example 51

1,1-Dioxopencillanoyloxymethyl 6-alpha-(Benzyloxycarbonylaminomethyl)penicillanate 1,1-Dioxide

Title product of Example 16 (0.5 g, 1.26 mmoles) was dissolved in 50 ml $CH_2Cl_2$. $H_2O$ (10 ml) was added and the pH adjusted to 8.6 with 1N NaOH. $NaHCO_3$ (0.106 g, 1.26 mmoles) and then tetrabutylammonium hydrogen sulfate (0.428 g, 1.26 mmoles) were added. The pH, which dropped to 5.0, was adjusted to 7.5 with 1N NaOH. After stirring 30 minutes at ambient temperature, the organic layer was separated, dried

($Na_2SO_4$) and concentrated *in vacuo* to yield tetrabutylammonium 6-alpha-(benzyloxycarbonylaminomethyl)penicillanate 1,1-dioxide as a foam. The latter was dissolved in 20 ml of acetone. Iodomethyl penicillanate 1,1-dioxide (prepared, for example, according to Godtfredsen *et al.,* U.S. Patent 4,342,772; 0.47 g) in 15 ml acetone was added and the mixture stirred for 5 minutes, and then concentrated *in vacuo*. The residue was slurried in 30 ml of ethyl acetate and crystalline tetrabutylammonium iodide (0.33 g) recovered by filtration. The filtrate was concentrated *in vacuo*. The residue was slurried in 30 ml of ethyl acetate and crystalline tetrabutylammonium iodide (0.33 g) recovered by filtration. The filtrate was concentrated *in vacuo* to yield title product as a foam, 0.82 g pnmr/$CDCl_3$/TMS/delta (ppm): 1.40 (3H, s), 1.42 (3H, s), 1.58 (6H, s), 3.41 (2H, m), 3.69 (3H, m), 4.40 (2H, s), 4.58 (2H, m), 5.08 (2H, s), 5.59 (1H, m), 5.86 (2H, s), 7.29 (5H, s).

Example 52

*p*-Toluenesulfonate Salt of 1,1-Dioxopenicillanoyloxymethyl 6-alpha-(Aminomethyl)penicillanate 1,1-Dioxide

Pd/C (10%, 1.2 g) was prehydrogenated in 10 ml $H_2O$. Pyridinium tosylate (0.482 g, 1.92 mmoles) and then a solution of title product of the preceding Example in 30 ml THF were added and the mixture hydrogenated at 50 psig for 1 hour. Catalyst was recovered by filtration over diatomaceous earth, with THF/$H_2O$ wash. THF was removed from the combined filtrate and washed by concentration *in vacuo*. The aqueous residue was extracted 1 × 30 ml ethyl acetate. The organic layer was dried ($Na_2SO_4$) and concentrated to yield 60 mg of solids. The aqueous layer was concentrated to 10 ml. Crystalline title product was recovered by filtration, 100 mg; mp 228—229°C (dec); pnmr/DMSO-$d_6$/delta (250 MHz): 1.35 (3H, s), 1.37 (3H, s), 1.49 (3H, s), 1.50 (3H, s), 2.29 (3H, s), 3.29 (1H, dd, J = 1.7, 16.6 Hz), 3.39 (2H, m), 3.72 (1H, dd, J = 4.6, 16.6 Hz), 3.92 (1H, m), 4.60 (1H, s), 4.77 (1H, s), 5.21 (2H, m), 5.96 (2H, s), 7.31 (4H, ABq).

Anal. Calcd. for $C_{18}H_{25}N_3O_{10}S_2.CH_3C_6H_4SO_3H$: C, 44.17; H, 4.89; N, 6.18
Found: C, 45.53; H, 4.76; N, 6.10.

The aqueous mother liquor was freeze dried to yield 160 mg solids. These were slurried in a small amount of water, filtered, washed with a small amount of ethyl acetate and dried to yield an additional 70 mg of purified title product, having identical mp and pnmr.

Example 53

Chloromethyl 6-alpha-(Benzyloxycarbonylaminomethyl)penicillanate 1,1-Dioxide

By the method of Example 49, title product of Example 16 (0.396 g, 1.0 mmole) was converted to its tetrabutylammonium salt. The latter was dissolved in 30 ml bromochloromethane, stirred at ambient temperature for 18 hours, concentrated *in vacuo* to a foam, and chromatographed on 50 g silica gel with 20% ethyl acetate/$CHCl_3$ as eluant in 20 ml fractions. Fractions 6—10 were combined and concentrated *in vacuo* to a foam, 0.25 g, $R_f$ 0.7 on tlc with same eluant; pnmr/$CDCl_3$/TMS/delta (ppm): 1.37 (3H, s), 1.54 (3H, s), 3.70 (3H, m), 4.38 (1H, s), 4.67 (1H, br s), 5.07 (2H, s), 5.66 (1H, d, J = 9 Hz), 5.70 (2H, ABq), 7.33 (5H, s).

Example 54

Iodomethyl 6-alpha-(Benzyloxycarbonyaminomethyl)penicillanate 1,1-Dioxide

Title product of the preceding Example (0.25 g, 0.563 mmole) was dissolved in 15 ml of acetone and purged with $N_2$. NaI (0.42 g, 2.8 mmoles) was added and the resulting solution stirred 17 hours and then concentrated *in vacuo*. The solids were triturated with $CHCl_3$, insolubles removed by filtration and title product recovered from filtrate by concentration *in vacuo* to a foam, 0.23 g, pnmr/$CDCl_3$/TMS/delta (ppm): 1.39 (3H, s), 1.55 (3H, s), 3.64 (3H, m), 4.28 (1H, s), 4.59 (1H, br. s), 5.04 (2H, s), 5.48 (1H, m), 5.83 (2H, ABq), 5.23 (5H, s).

Example 55

Methylene *bis*-[6-alpha-(Benzyloxycarbonylaminomethyl)-1,1-dioxopenicillanate]

Title product of Example 16 (0.17 g, 0.429 mmole) was converted to its tetrabutylammonium salt (0.27 g) according to the procedure of Example 49. The latter was dissolved in 10 ml acetone and added to a solution of title product of the preceding Example (0.23 g, 0.429 mmole) in 10 ml acetone. The mixture was stirred 15 minutes, concentrated to a foam *in vacuo* and the foam slurried in 20 ml ethyl acetate. The slurry was filtered to yield tetrabutylammonium iodide (110 mg) and the filtrate concentrated *in vacuo* to yield title product as a foam, 0.28 g, pnmr/$CDCl_3$/TMS/delta (ppm): 1.36 (6H, s), 1.52 (6H, s), 3.73 (6H, m), 4.40 (2H, s), 4.69 (2H, br. s), 5.08 (4H, s), 5.77 (4H, m), 7.28 (10H, s).

Example 56

*bis*-(*p*-Toluenesulfonate) Salt of Methylene *bis*-[6-alpha-(Aminomethyl)-1,1-dioxopenicillanate]

By the procedure of Example 52, title product of the preceding Example was hydrogenated. After recovery of catalyst and removal of THF, the aqueous residue was extracted 3 × 20 ml ethyl acetate and freeze dried to yield title product, 0.19 g, pnmr/DMSO-$d_6$/TMS/delta (250 MHz): 1.37 (6H, s), 1.50 (6H, s), 2.31 (6H, s), 3.40 (4H, m), 3.94 (2H, m), 4.77 (2H, s), 5.30 (2H, m), 5.98 (2H, m), 7.32 (8H, ABq).

**Claims for the Contracting States: BE CH DE FR GB IT LI LU NL SE**

1. A compound having the stereochemical formula

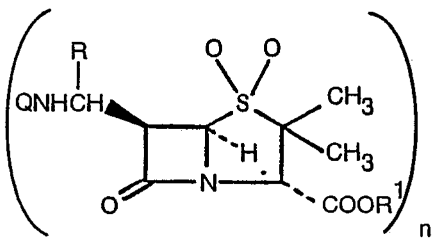

(A) or

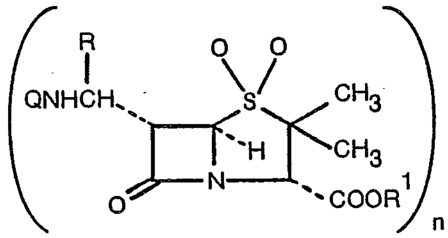

(B)

wherein in a first alternative

n is 1;

R is hydrogen;

Q is benzyl, *o-*, *m-* or *-p*-hydroxybenzyl, phenethyl, or 2-, 3- or 4-picolyl; and

$R^1$ is hydrogen, or a radical group forming an ester which is hydrolyzable under physiological conditions;

and in a second alternative

Q is hydrogen;

R is hydrogen or methyl; and

n is 2 and $R^1$ is $—CH_2—$; or

n is 1 and $R^1$ is hydrogen, a radical group forming an ester hydrolyzable under physiological conditions; 1,1-dioxopenicillanoyloxymethyl; or $R^4$ wherein $R^4$ is

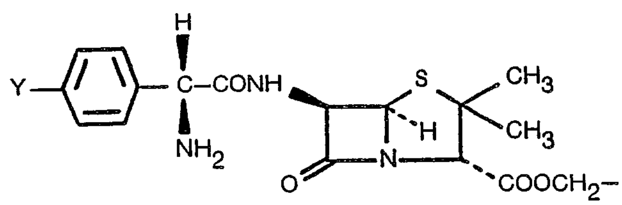

wherein Y is hydrogen, hydroxy, $(C_2—C_7)$-alkanoyloxy, $(C_2—C_7)$-alkoxycarbonyloxy, benzoyloxy, or benzoyloxy monosubstituted with $(C_1—C_4)$alkyl, $(C_1—C_4)$-alkoxy or halo;
a pharmaceutically-acceptable acid addition salt thereof; or a pharmaceutically-acceptable cationic salt thereof when $R^1$ is hydrogen.

2. A compound of claim 1 in the second alternative, wherein n is 1, $R^1$ is hydrogen and R is hydrogen or methyl.

3. A compound of claim 1 in the second alternative, wherein n is 1 and $R^1$ is pivaloyloxymethyl.

4. A compound of claim 3 which is in the form of its *P*-toluenesulfonate salt.

5. A compound of claim 1 in the second alternative, wherein n is 1 and $R^1$ is 1,1-dioxopenicillanoyloxymethyl.

6. A compound of claim 1 in the second alternative, wherein n is 2 and $R^1$ is $—CH_2—$.

7. A compound of claim 1 in the first alternative.

8. A compound of the stereochemical formula

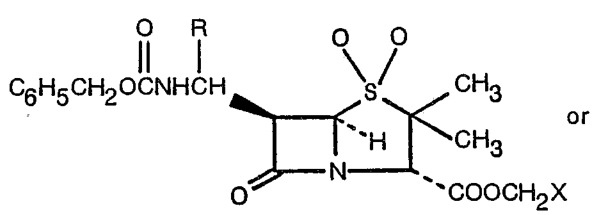

or

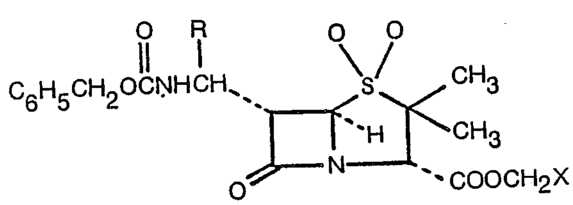

wherein R is hydrogen or methyl and X is chloro or iodo.

9. A compound of the stereochemical formula

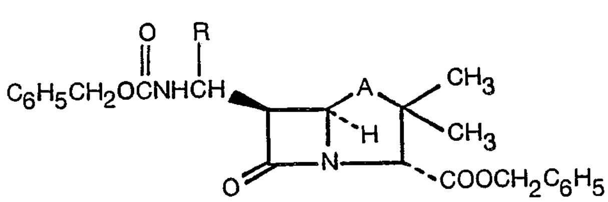

, (V)

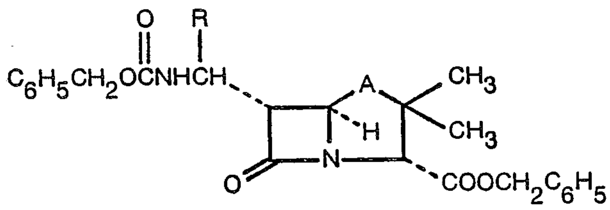

(VI)

wherein R is hydrogen or methyl, and A is

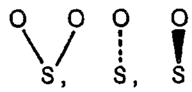 or S;

10. A compound of the stereochemical formula

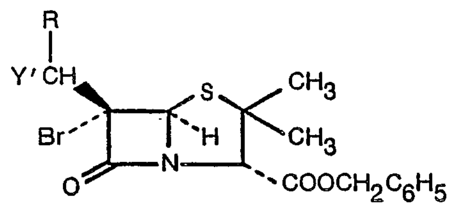

or

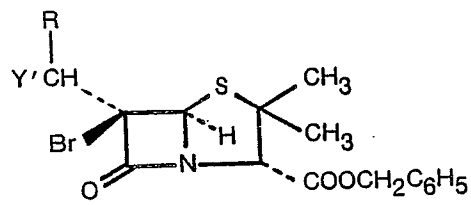

wherein

R is hydrogen or methyl and Y′ is
benzyloxycarbonylamino,
amino,
azido, or
trifluoromethanesulfonyloxy.

11. A pharmaceutical composition for treating bacterial infections which comprises in a weight ratio of 1:3 to 3:1 a compound of claim 1 excluding compounds of the second alternative wherein Q is hydrogen, R is hydrogen or methyl, n is I and $R^1$ is $R^4$; and a beta-lactam antibiotic.

12. A pharmaceutical composition according to claim 11 wherein the beta-lactam antibiotic is:
amoxicillin,
ampicillin,
azlocillin,
bacampicillin,
carbenicillin,
carbenicillin indanyl,
carbenicillin phenyl,
cefaclor,
cefadroxil,
cefaloram,
cefamandole,
cefamandole nafate,
cefaparole,
cefatrizine,
cefazolin,
cefmenoxime
cefonicid
cefodizime
cefoperazone,
ceforanide,
cefotaxime,
cefoxitin,
cefsulodin,
ceftazidime,
ceftizoxime,
ceftriaxone,
cefuroxime
cephacetrile
cephalexin,
cephaloglycin,

cephaloridine,
cephalothin,
cephapirin,
cephradine,
cyclacillin,
epicillin,
hetacillin,
levopropylcillin,
mecillinam,
mezlocillin,
penicillin G,
penicillin V,
phenethicillin,
piperacillin,
pirbenicillin,
pivampicillin,
sarmoxicillin,
sarpicillin,
suncillin,
talampicillin or
ticarcillin; or

a pharmaceutically acceptable salt thereof.

13. A pharmaceutical composition suitable for treating a bacterial infection in a mammal which comprises an antibacterially effective amount of a compound of claim 1 in the second alternative wherein Q is hydrogen, R is hydrogen or methyl, n is 1 and $R^1$ is $R^4$, together with a pharmaceutically acceptable diluent or carrier.

**Claims for the Contracting State: AT**

1. A process for preparing a compound having the stereochemical formula

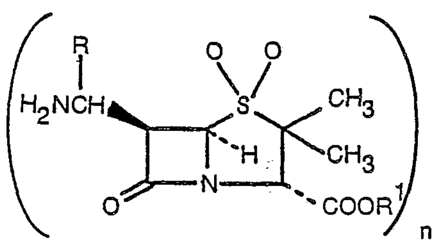
(I)

or

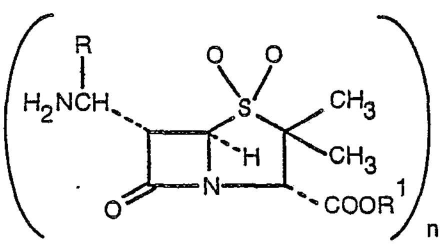
(II)

wherein

R is hydrogen or methyl; and

n is 2 and $R^1$ is $—CH_2—$; or

n is 1 and $R^1$ is hydrogen, a radical group forming an ester hydrolyzable under physiological conditions; 1,1-dioxopenicillanoyloxymethyl; or $R^4$ wherein $R^4$ is

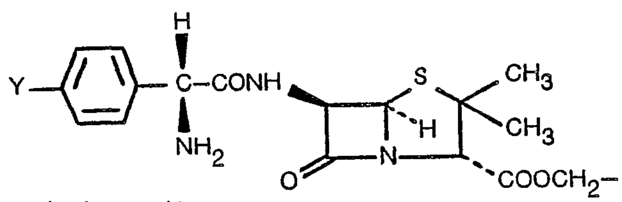

wherein Y is hydrogen, hydroxy, $(C_2—C_7)$-alkanoyloxy, $(C_2—C_7)$-alkoxycarbonyloxy, benzoyloxy, or benzoyloxy monosubstituted with $(C_1—C_4)$-alkyl, $(C_1—C_4)$-alkoxy or halo; which is characterized by hydrogenolysis, in a reaction inert solvent over a noble metal catalyst, of a compound of the formula

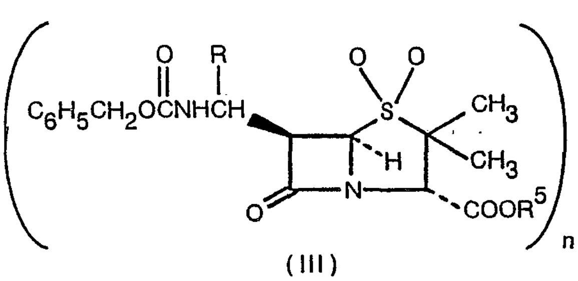
(III)

or

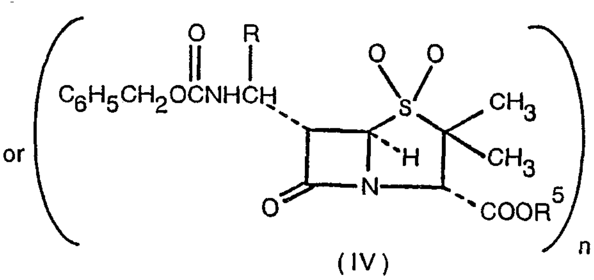
(IV)

wherein

R is hydrogen or methyl; and

n is 2 and $R^5$ is —$CH_2$—; or

n is 1 and $R^5$ is benzyl, a radical group forming an ester hydrolyzable under physiological conditions; 1,1-dioxopenicillanoyloxymethyl; or

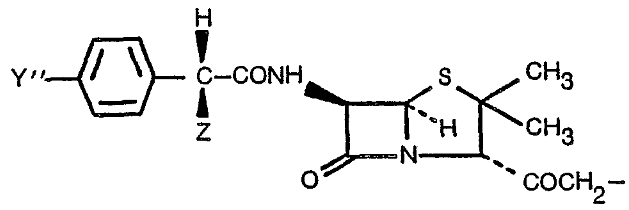

(C)

wherein Y″ is hydrogen, benzyloxycarbonyl, ($C_2$—$C_7$)-alkanoyloxy, ($C_2$—$C_7$)-alkoxycarbonyloxy, benzoyloxy, or benzoyloxy monosubstituted with ($C_1$—$C_4$)-alkyl, ($C_1$—$C_4$)-alkoxy or halo; and Z is azido, or benzyloxycarbonylamino; and, if desired, converting said compound of the formula (I) or (II) to a pharmaceutically acceptable acid addition salt, or to a pharmaceutically acceptable cationic salt when $R^1$ is hydrogen.

2. A process of claim 1 which is further characterized by the preparation of the compound of the formula (III) or (IV) wherein R is hydrogen or methyl, n is 2 and $R^5$ is —$CH_2$— by reacting a compound of the formula

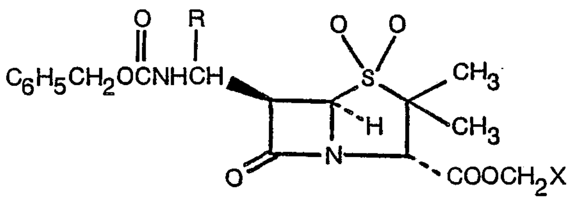

(V)

or

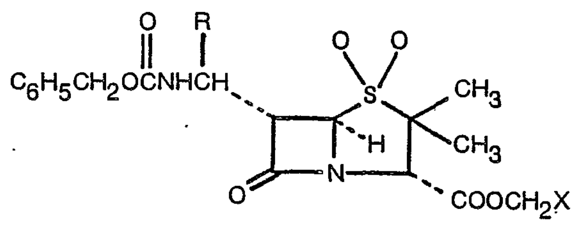

(VI)

wherein R is hydrogen or methyl and X is chloro or bromo, with a salt of an acid of the formula

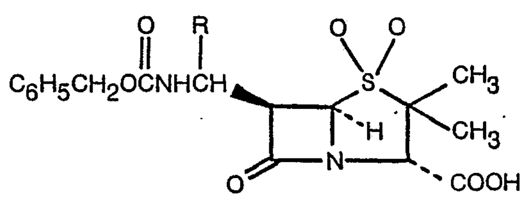

(VII)

or

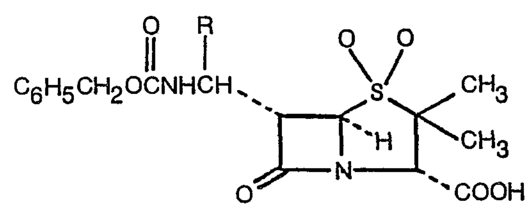

(VIII)

3. A process of claim 1 which is further characterized by the preparation of the compound of the formula (III) or (IV) wherein R is hydrogen or methyl, n is 1 and $R^5$ is benzyl, by one step or stepwise oxidation of a sulfide of the formula

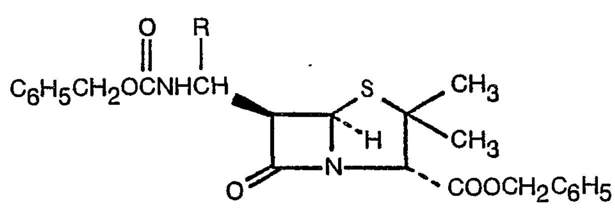

(IX)

or

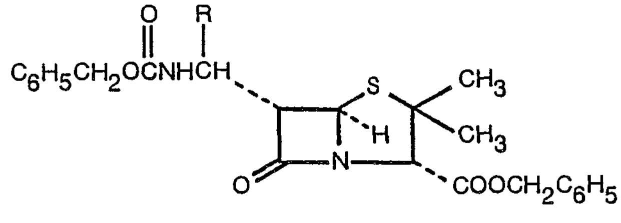

,

(X)

wherein R is hydrogen or methyl.

4. A process of claim 1 which is further characterized by the preparation of the compound of the formula (III) or (IV) wherein R is hydrogen or methyl, n is 1 and $R^5$ is a radical group forming an ester hydrolyzable under physiological conditions selected from the group consisting of pivaloyloxymethyl and acetoxymethyl, by reacting chloromethyl pivalate or bromomethyl acetate with a salt of an acid of the formula (VII) or (VIII).

5. A process of claim 1 which is further characterized by preparation of the compound of the formula (III) or (IV) wherein R is hydrogen or methyl, n is 1 and $R^5$ is 1,1-dioxopenicillanoyloxymethyl by either reacting the halomethyl ester of the formula (V) or (VI) with a salt of penicillanic acid 1,1-dioxide; or reacting the salt of an acid of the formula (VII) or (VIII) with a halomethyl ester of penicillanic acid.

6. A process of claim 1 which is further characterized by the preparation of the compound of the formula (III) or (IV) wherein R is hydrogen or methyl, n is 1 and $R^5$ is the radical of the formula (C) by either reacting the iodomethyl ester of the formula (V) or (VI) with a salt of an acid of the formula

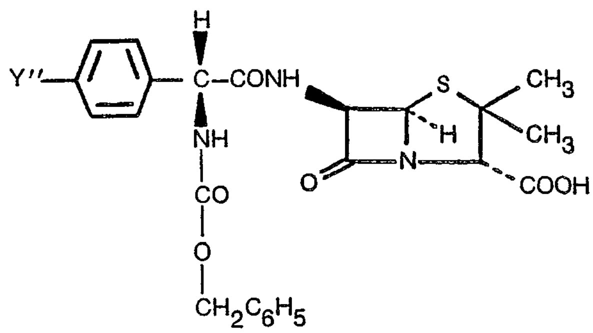

(XI)

wherein Y'' is H, benzyloxycarbonyloxy, ($C_2$—$C_7$)-alkoxycarbonyloxy, benzoyloxy, or benzoyloxy monosubstituted with ($C_1$—$C_4$)alkyl, ($C_1$—$C_4$)alkoxy or halo; or reacting the salt of the acid of the formula (VII) or (VIII) with a halomethyl ester of the acid of the formula (XI).

7. A process for preparing a compound of the formula

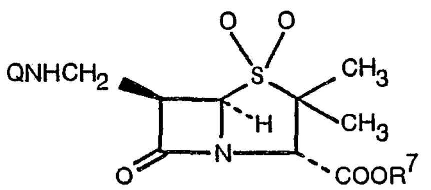

(XII) or

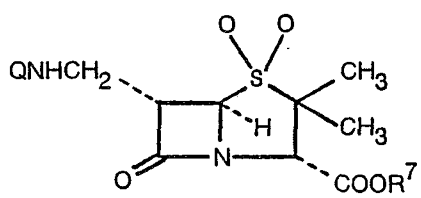

(XIII)

wherein

Q is benzyl, *o*-, *m*- or -*p*-hydroxybenzyl, phenethyl, or 2-, 3- or 4-picolyl; and

$R^7$ is hydrogen, or a radical group forming an ester which is hydrolyzable under physiological conditions;

which is characterized by reacting an aminomethyl compound of the formula

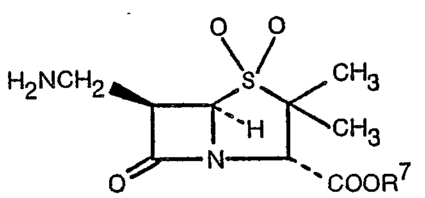

or

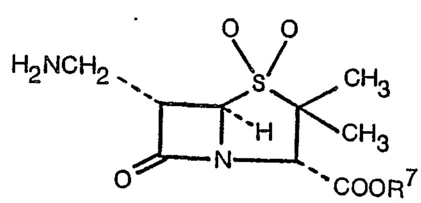

wherein $R^7$ is as defined above, with an equivalent of benzaldehyde, *o*-, *m*- or *p*-hydroxybenzaldehyde, phenylacetaldehyde or 2-, 3- or 4-pyridinecarbaldehyde in the presence of excess sodium cyanoborohydride; and, if desired, converting said compound of the formula (XII) or (XIII) to a pharmaceutically-acceptable acid addition salt; or converting a compound of the formula (XII) or (XII) wherein $R^7$ is hydrogen to a pharmaceutically acceptable cationic salt.

8. A process for the preparation of a compound of the formula

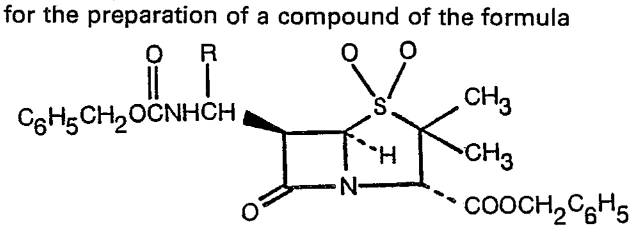

or

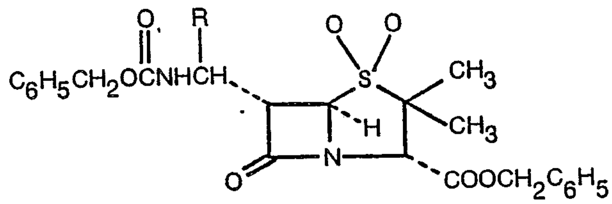

which is characterized by one step or stepwise oxidation of a compound of the formula

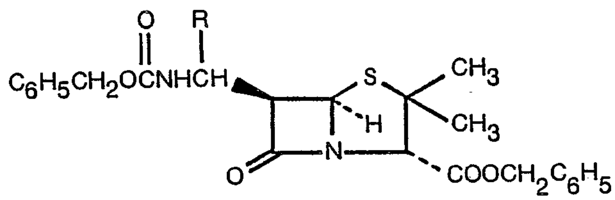

or

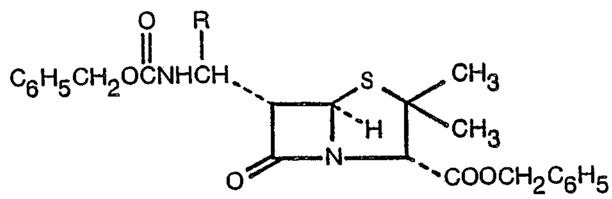

9. A process for the preparation of a compound of the formula

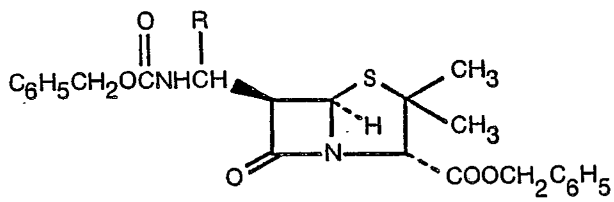

or

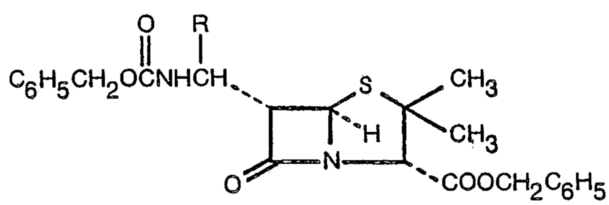

which is characterized by the steps of

(a) reacting an alcohol of the formula

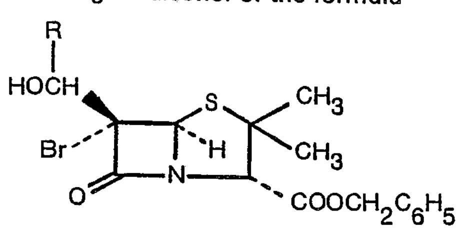

or

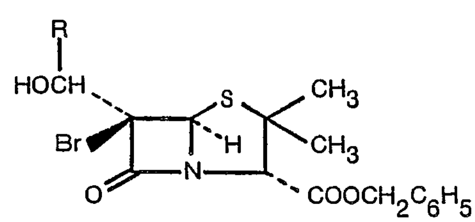

with trifluoromethanesulfonyl chloride to form trifluoromethanesulfonate ester of the formula

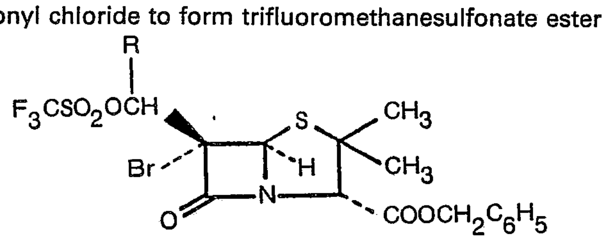

or

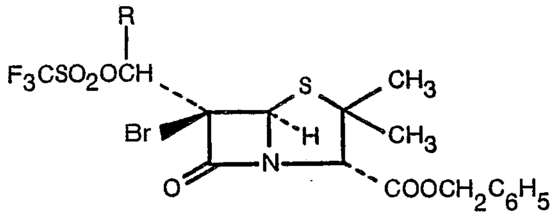

;

(b) reacting said trifluoromethanesulfonate ester with an azide salt to form an azido compound of the formula

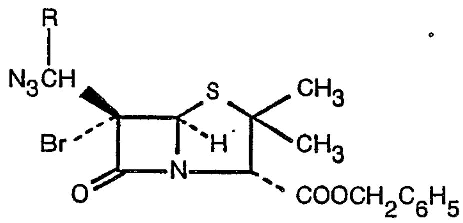

or

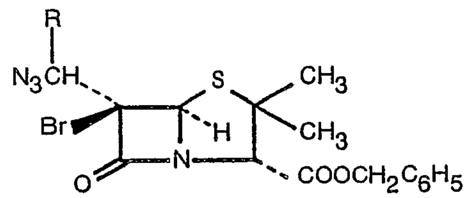

;

(c) reducing said azido compound with hydrogen sulfide to form an amino bromo compound of the formula

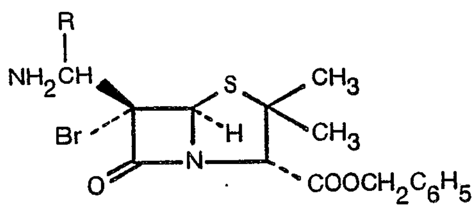

or

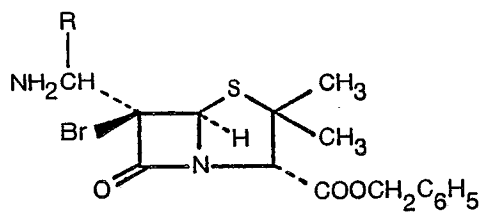

;

(d) reacting said amino bromo compound with benzyl chloroformate to form an amido bromo compound of the formula

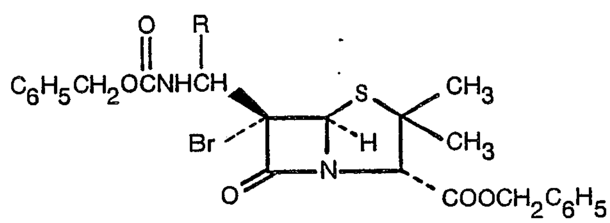

or

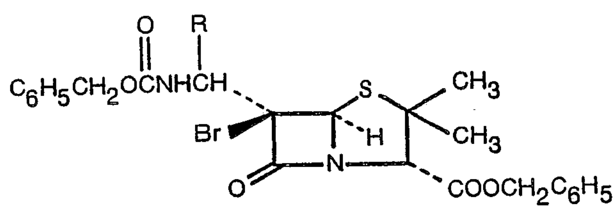

; and

(e) debrominating said amido bromo compound with tri($n$-butyl)tin hydride.

**Patentansprüche für die Vertragsstaaten: BE CH DE FR GB IT LI LU NL SE**

1. Verbindung mit der stereochemischen Formel

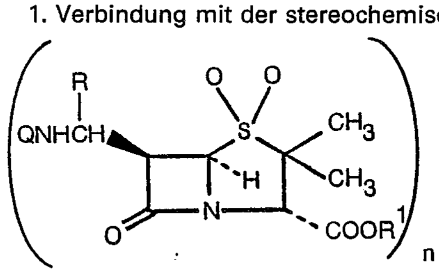

(A)

oder

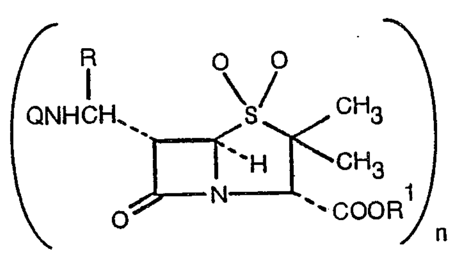

(B)

worin als erste Möglichkeit

n = 1 ist

R Wasserstoff ist,

Q Benzyl, o-, m- oder p-Hydroxybenzyl, Phenethyl oder 2-, 3- oder 4-Picolyl ist und

$R^1$ Wasserstoff oder ein einen Ester, der unter physiologischen Bedingungen hydrolysierbar ist, bildender Rest ist,

und als zweite Möglichkeit

Q Wasserstoff ist,

R Wasserstoff oder Methyl ist und

n = 2 ist und $R^1$ $-CH_2-$ ist oder

n = 1 ist und $R^1$ Wasserstoff,

ein einen unter physiologischen Bedinungen hydrolysierbaren Ester bildender Rest, 1,1-Dioxopenicillanoyloxymethyl oder $R^4$ ist, wobei $R^4$

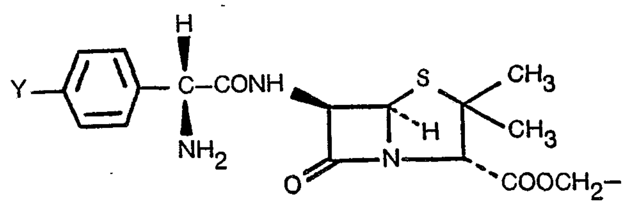

ist, worin Y Wasserstoff, Hydroxy, $(C_2-C_7)$-Alkanoyloxy, $(C_2-C_7)$-Alkoxycarbonyloxy, Benzoyloxy oder mit $(C_1-C_4)$-Alkyl, $(C_1-C_4)$-Alkoxy oder Halogen monosubstituiertes Benzoyloxy ist, ein pharmazeutisch annehmbares Säureadditionssalz hiervon oder ein pharmazeutisch annhembares kationisches Salz hiervon, wenn $R^1$ Wasserstoff ist.

2. Verbindung nach Anspruch 1 in der zweiten Möglichkeit, worin n gleich 1 ist, $R^1$ Wasserstoff ist und R Wasserstoff oder Methyl ist.

3. Verbindung nach Anspruch 1 in der zweiten Möglichkeit, worin n gleich 1 ist und $R^1$ Pivaloyloxymethyl ist.

4. Verbindung nach Anspruch 3, die in der Form ihres p-Toluolsulfonatsalzes vorliegt.

5. Verbindung nach Anspruch 1 in der zweiten Möglichkeit, worin n gleich 1 ist und $R^1$ 1,1-Dioxopenicillanoyloxymethyl ist.

6. Verbindung nach Anspruch 1 in der zweiten Möglichkeit, worin n gleich 2 ist und $R^1$ $-CH_2-$ ist.

7. Verbindung nach Anspruch 1 in der ersten Möglichkeit.

8. Verbindung mit der stereochemischen Formel

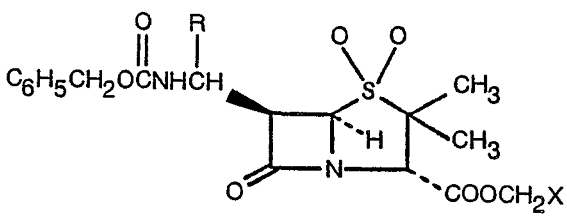

oder

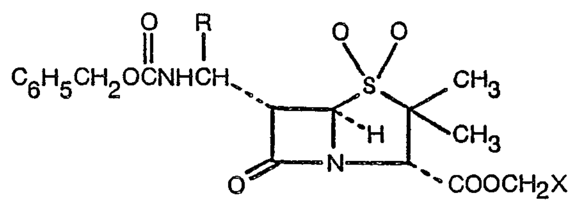

worin R Wasserstoff oder Methyl ist und X Chlor oder Jod ist.

9. Verbindung mit der stereochemischen Formel

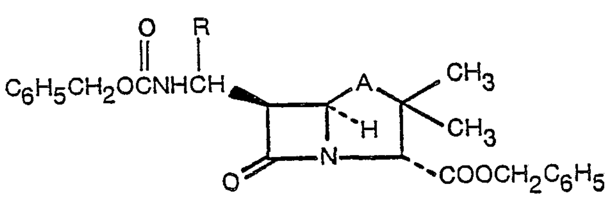

oder

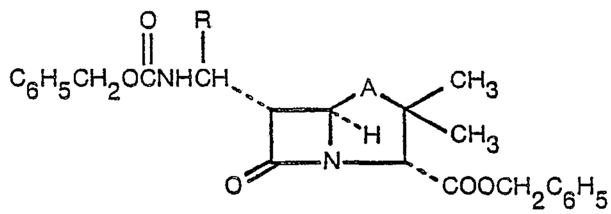

worin R Wasserstoff oder Methyl ist und A

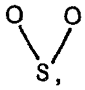, 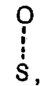, 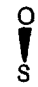

oder S ist.

10. Verbindung mit der stereochemischen Formel

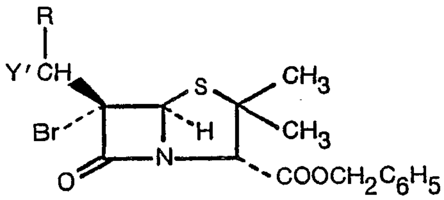

oder

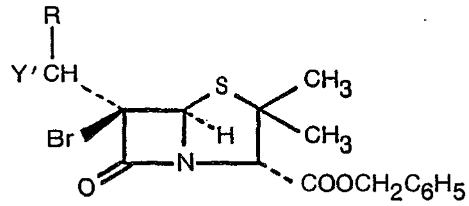

worin R Wasserstoff oder Methyl ist und Y' Benzyloxycarbonylamino, Amino, Azido oder Trifluormethansulfonyloxy ist.

11. Pharmazeutische Zusammensetzung zur Behandlung bakterieller Infektionen, die in einem Gewichtsverhältnis von 1:3 bis 3:1 eine Verbindung nach Anspruch 1, ausgenommen Verbindungen der zweiten Möglichkeit, worin Q Wasserstoff ist, R Wasserstoff oder Methyl ist, n gleich 1 ist und $R^1$ $R^4$ ist, und ein β-Lactam-antibiotikum enthält.

12. Pharmazeutische Zusammensetzung nach Anspruch 11, worin das β-Lactam-antibiotikum

Amoxicillin,
Ampicillin,
Azlocillin,
Bacampicillin,
Carbenicillin,
Carbenicillin-Indanyl,
Carbenicillin-Phenyl,
Cefaclor,
Cefadroxil,
Cefaloram,
Cefamandol,
Cefamandol-Nafat,
Cefaparol,
Cefatrizin,
Cefazolin,
Cefmenoxim,
Cefonicid,
Cefodizim,
Cefoperazon,
Ceforanid,
Cefotaxim,
Cefoxitin,
Cefsulodin,
Ceftazidim,
Ceftizoxim,
Ceftriaxon,
Cefuroxim,
Cephacetril,
Cephalexin,
Cephaloglycin,
Cephaloridin,
Cephalothin,
Cephapirin,
Cephradin,
Cyclacillin,
Epicillin,
Hetacillin,
Levopropylcillin,
Mecillinam,
Mezlocillin,
Penicillin G,

Penicillin V,
Phenethicillin,
Piperacillin,
Pirbenicillin,
Pivampicillin,
Sarmoxicillin,
Sarpicillin,
Suncillin,
Talampicillin oder,
Ticarcillin ist, oder

ein pharmazeutisch annehmbares Salz hiervon.

13. Pharmazeutische Zusammensetzung, die zur Behandlung einer bakteriellen Infektion in einem Säuger geeignet ist und eine antibakteriell wirksame Menge einer Verbindung nach Anspruch 1 in der zweiten Möglichkeit, worin Q Wasserstoff ist, R Wasserstoff oder Methyl ist, n gleich 1 und $R^1$ gleich $R^4$ ist, zusammen mit einem pharmazeutisch annehmbaren Verdünnungsmittel oder Träger enthält.

**Patentansprüche für den Vertragsstaat: AT**

1. Verfahren zur Herstellung einer Verbindung der stereochemischhen Formel

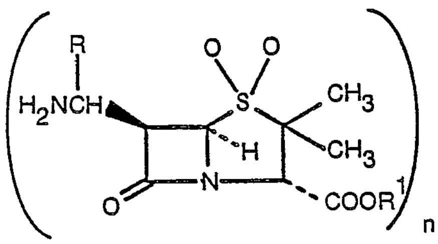
(I)
oder
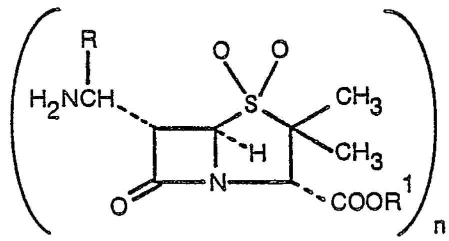
(II)

worin

R Wasserstoff oder Methyl ist und
n gleich 2 und $R^1$ —$CH_2$— ist, oder
n gleich 1 und $R^1$ Wasserstoff,

ein einen unter physiologischen Bedingungen hydrolysierbaren Ester bildender Rest, 1,1-Dioxopenicillanoyloxymethyl oder $R^4$ ist, wobei $R^4$

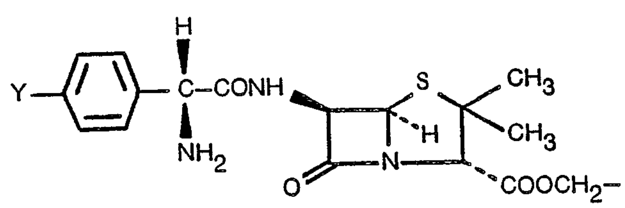

ist, worin Y Wasserstoff, Hydroxy, ($C_2$—$C_7$)-Alkanoyloxy, ($C_2$—$C_7$)-Alkoxycarbonyloxy, Benzoyloxy oder durch ($C_1$—$C_4$)-Alkyl, ($C_1$—$C_4$)-Alkoxy oder Halogen monosubstituiertes Benzoyloxy ist, gekennzeichnet durch Hydrogenolyse einer Verbindung der Formel

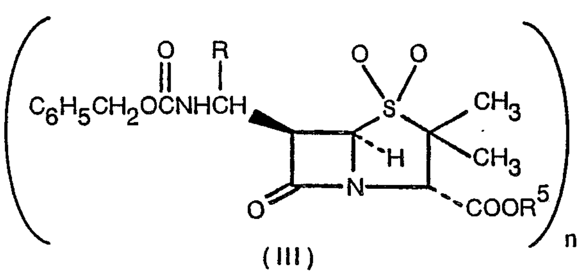
(III)
oder
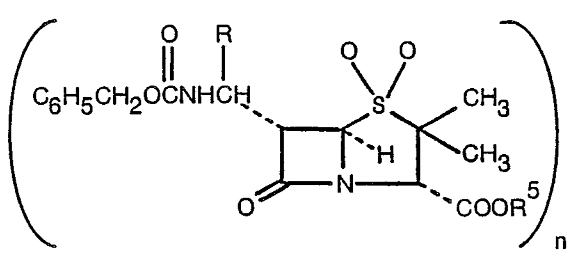
(IV)

worin

R Wasserstoff oder Methyl ist und
n gleich 2 ist und $R^5$ —$CH_2$— ist, oder
n gleich 1 ist und $R^5$ Benzyl,

ein einen unter physiologischen Bedingungen hydrolysierbaren Ester bildender Rest, 1,1-Dioxopenicillanoyloxymethyl oder

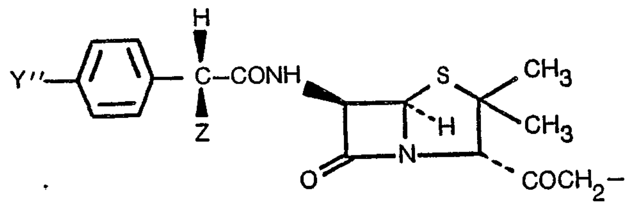

(C)

ist, worin Y" Wasserstoff, Benzyloxycarbonyloxy, $(C_2—C_7)$-Alkanoyloxy, $(C_2—C_7)$-Alkoxycarbonyloxy, Benzoyloxy oder durch $(C_1—C_4)$-Alkyl, $(C_1—C_4)$-Alkoxy oder Halogen monosubstituiertes Benzoyloxy ist und Z Azido oder Benzyloxycarbonylamino ist, in einem reaktionsinerten Lösungsmittel über einem Edelmetallkatalysator und, wenn gewünscht, Umwandeln der genannten Verbindung der Formel (I) oder (II) in ein pharmazeutisch annehmbares Säureadditionssalz oder in ein pharmazeutisch annehmbares kationisches Salz, wenn $R^1$ Wasserstoff ist.

2. Verfahren nach Anspruch 1, gekennzeichnet ferner durch die Herstellung der Verbindung der Formel (III) oder (IV), worin R Wasserstoff oder Methyl ist, n 2 und $R^5$ $—CH_2—$ ist, durch Umsetzen einer Verbindung der Formel

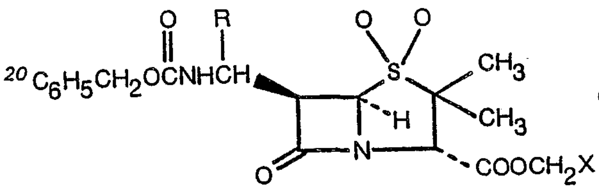

(V)

oder

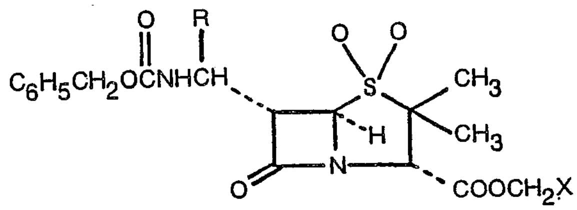

(VI)

worin R Wasserstoff oder Methyl und X Chlor oder Brom ist, mit einem Salz einer Säure der Formel

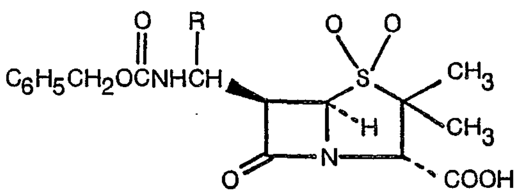

(VII)

oder

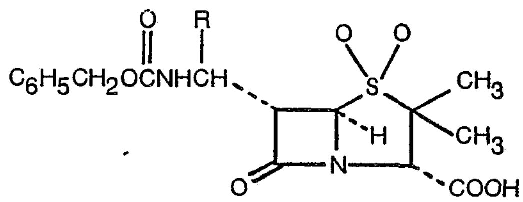

(VIII)

3. Verfahren nach Anspruch 1, gekennzeichnet ferner durch die Herstellung der Verbindung der Formel (III) oder (IV), worin R Wasserstoff oder Methyl, n 1 und $R^5$ Benzyl ist, durch einstufige oder stufenweise Oxidation eines Sulfids der Formel

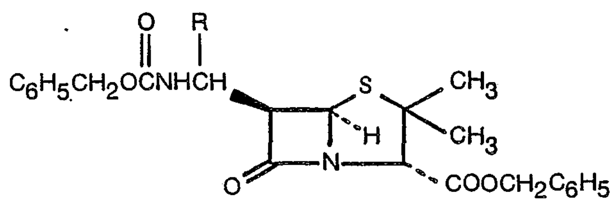

(IX)

oder

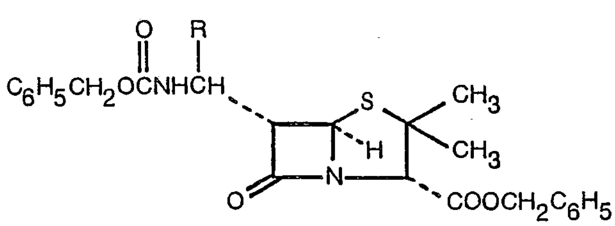

(X)

worin R Wasserstoff oder Methyl ist.

4. Verfahren nach Anspruch 1, gekennzeichnet ferner durch die Herstellung der Verbindung der Formel (III) oder (IV), worin R Wasserstoff oder Methyl, n 1 und $R^5$ ein einen unter physiologischen Bedingungen hydrolysierbaren Ester bildender Rest, ausgewählt unter Pivaloyloxymethyl und Acetoxymethyl, ist, durch Umsetzen von Chlormethylpivalat oder Brommethylacetat mit einem Salz einer Säure der Formel (VII) oder (VIII).

5. Verfahren nach Anspruch 1, gekennzeichnet ferner durch die Herstellung der Verbindung der Formel (III) oder (IV), worin R Wasserstoff oder Methyl, n 1 und $R^5$ 1,1-Dioxopenicillanoyloxymethyl ist, entweder durch Umsetzen des Halogenmethylesters der Formel (V) oder (VI) mit einem Salz von Penicillinsäure-1,1-dioxid oder durch Umsetzen des Salzes einer Säure der Formel (VII) oder (VIII) mit einem Halogenmethylester der Penicillinsäure.

6. Verfahren nach Anspruch 1, gekennzeichnet ferner durch die Herstellung der Verbindung der Formel (III) oder (IV), worin R Wasserstoff oder Methyl, n 1 und $R^5$ der Rest der Formel (C) ist, entweder durch Umsetzen des Jodmethylesters der Formel (V) oder (VI) mit einem Salz einer Säure der Formel

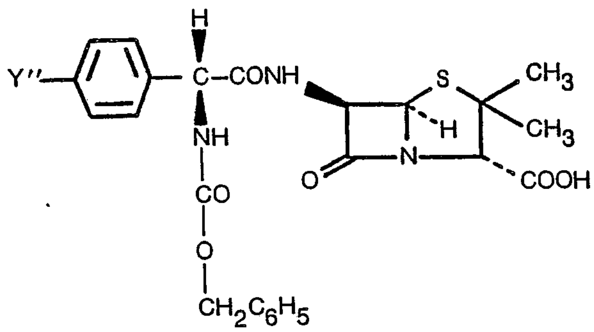

(XI)

worin Y″ H, Benzyloxycarbonyloxy, $(C_2—C_7)$Alkoxycarbonyloxy, Benzoyloxy oder durch $(C_1—C_4)$Alkyl, $(C_1—C_4)$Alkoxy oder Halogen monosubstituiertes Benzoyloxy ist, oder durch Umsetzen des Salzes der Säure der Formel (VII) oder (VIII) mit einem Halogenmethylester der Säure der Formel (XI).

7. Verfahren zur Herstellung einer Verbindung der Formel

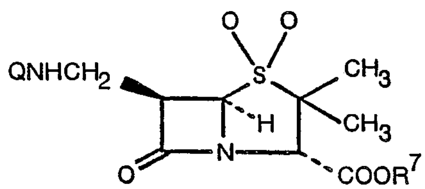

(XII) oder

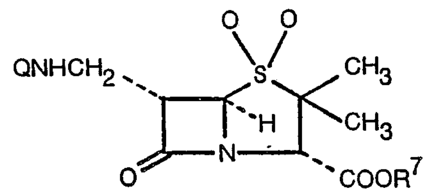

(XIII)

worin

Q Benzyl, o-, m- oder p-Hydroxybenzyl, Phenethyl oder 2-, 3- oder 4-Picolyl ist und

$R^7$ Wasserstoff oder ein einen unter physiologischen Bedingungen hydrolysierbaren Ester bildender Rest ist, gekennzeichnet durch Umsetzen einer Aminomethyl-Verbindung der Formel

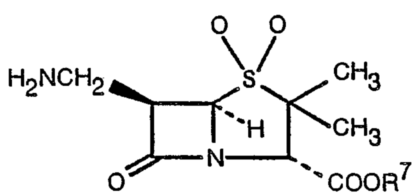

oder

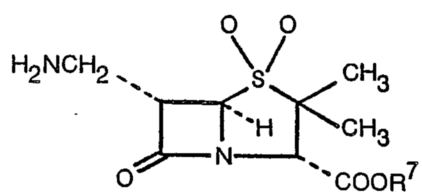

worin $R^7$ wie oben definiert ist, mit einem Äquivalent Benzaldehyd, o-, m- oder p-Hydroxybenzaldehyd, Phenylacetaldehyd oder 2-, 3- oder 4-Pyridincarbaldehyd in Gegenwart von überschüssigem Natriumcyanoborhydrid und, wenn gewünscht, Umwandeln der Verbindung der Formel (XII) oder (XIII) in ein pharmazeutisch annehmbares Säureadditionssalz oder Umwandeln einer Verbindung der Formel (XII) oder (XIII), worin $R^7$ Wasserstoff ist, in ein pharmazeutich annehmbares kationisches Salz.

8. Verfahren zur Herstellung einer Verbindung der Formel

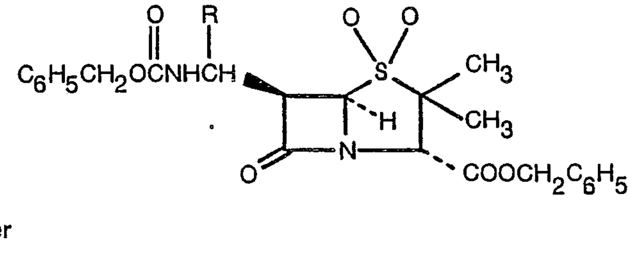

oder

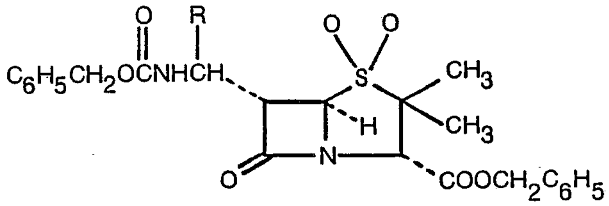

gekennzeichnet durch eine einstufige oder stufenweise Oxidation einer Verbindung der Formel

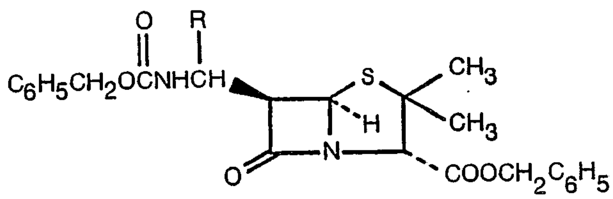

oder

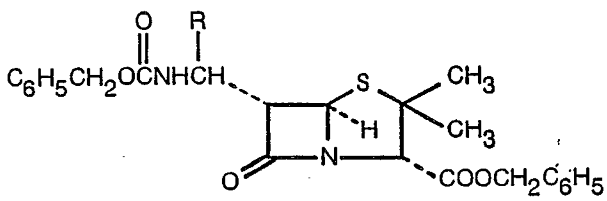

9. Verfahren zur Herstellung einer Verbindung der Formel

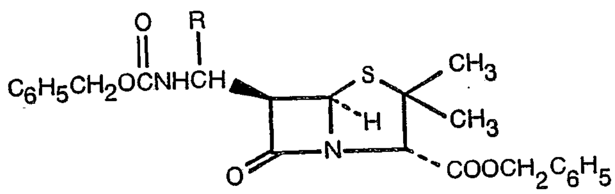

oder

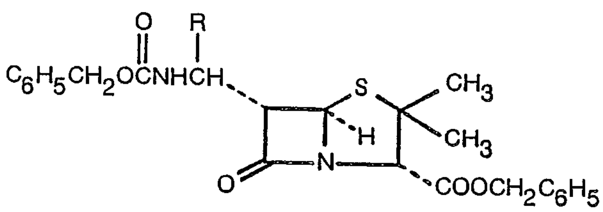

gekennzeichnet durch die Stufen

a) Umsetzen eines Alkohols der Formel

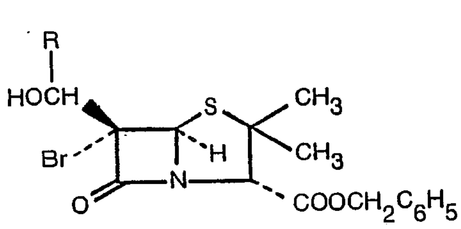

oder

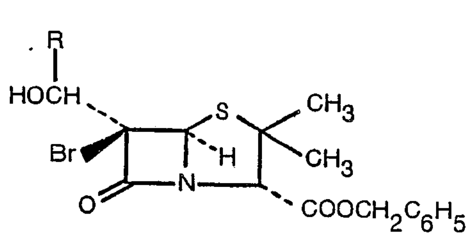

mit Trifluormethansulfonylchlorid zu Trifluormethansulfonatester der Formel·

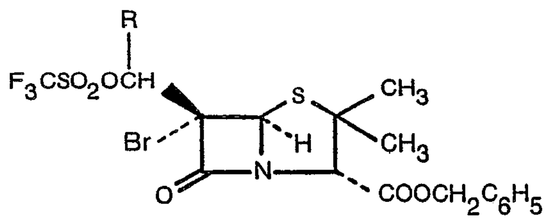

oder

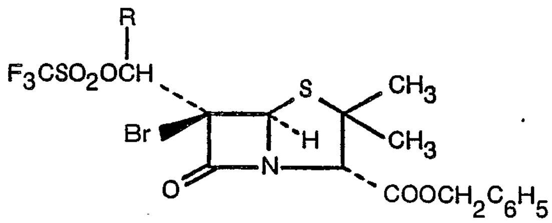

;

b) Umsetzen des genannten Trifluormethansulfonatesters mit einem Azidsalz zu einer Azidoverbindung der Formel

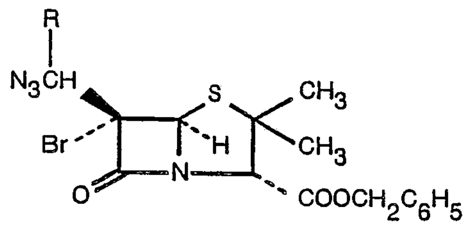

oder

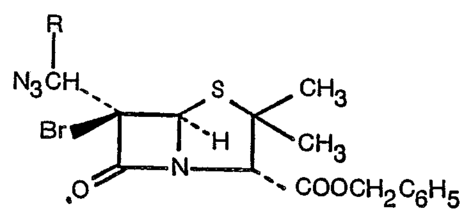

;

c) Reduzieren der genannten Azidoverbindung mit Schwefelwasserstoff zu einer Aminobromverbindung der Formel

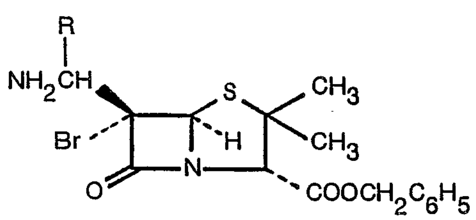

oder

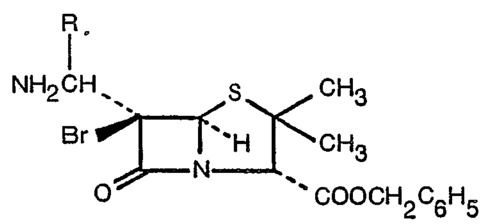

;

d) Umsetzen der Aminobromverbindung mit Benzylchlorformiat zu einer Amidobromverbindung der Formel

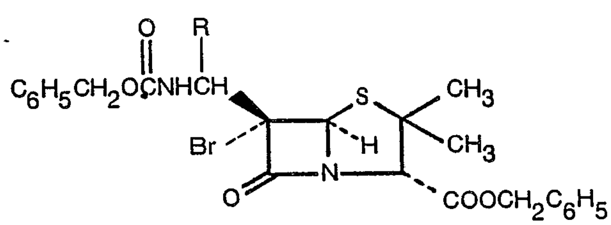

oder

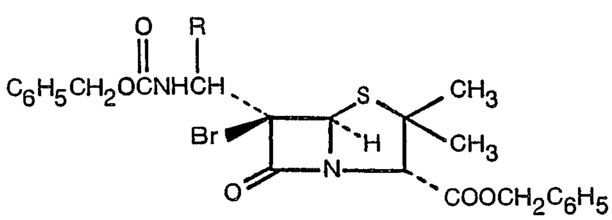

;

und

e) Debromieren der Amidobromverbindung mit Tri-(n-butyl)zinnhydrid.

**Revendications pour les Etats contractants: BE CH DE FR GB IT LI LU NL SE**

1. Composé répondant à la formule stéréochimique

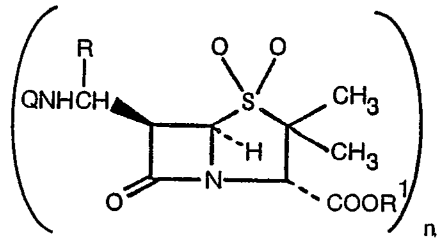

(A) ou

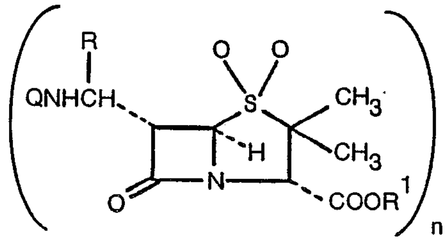

(B)

dans laquelle, en première variante,

n est égal à 1;

R est l'hydrogène;

Q est le groupe benzyle, *o*-, *m*- ou -*p*-hydroxybenzyle, phénéthyle ou 2-, 3- ou 4-picolyle; et

$R^1$ est l'hydrogène ou un groupe radicalaire formant un ester qui est hydrolysable dans des conditions physiologiques;

et en second variante

Q représente l'hydrogène;

R est l'hydrogène ou le groupe méthyle; et

n est égal à 2 et $R^1$ représente $-CH_2-$; ou bien

n est égal à 1 et $R^1$ est l'hydrogene,

un groupe radicalaire formant un ester hydrolysable dans des conditions physiologiques; le groupe 1,1-dioxopénicillanoyloxyméthyle; ou bien $R^4$, le groupe $R^4$ répondant à la formule

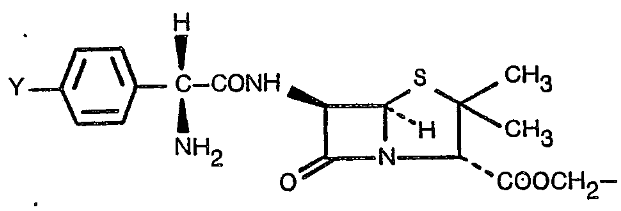

dans laquelle Y est l'hydrogène, un groupe hydroxy, un groupe alcanoyloxy en $C_2$ à $C_7$, un groupe alkoxycarbonyloxy en $C_2$ à $C_7$, un groupe benzoyloxy, ou un groupe benzoyloxy monosubstitué avec un substituant alkyle en $C_1$ à $C_4$, alkoxy en $C_1$ à $C_4$ ou halogéno;

un sel d'addition d'acide pharmaceutiquement acceptable de ce composé; ou un sel cationique pharmaceutiquement acceptable de ce composé lorsque $R^1$ est l'hydrogène.

2. Composé suivant la revendication 1, dans la seconde variante duquel n est égal à 1, $R^1$ est l'hydrogène et R est l'hydrogène ou le groupe méthyle.

3. Composé suivant la revendication 1, dans la seconde variante duquel n est égal ax 1 et $R^1$ est un groupe pivaloyloxyméthyle.

4. Composé suivant la revendication 3, qui est sous la forme de son sel d'acide p-toluènesulfonique.

5. Composé suivant la revendication 1, dans la seconde variante duquel n est égal à 1 et $R^1$ est un groupe 1,1-dioxopénicillanoyloxyméthyle.

6. Composé suivant la revendication 1, dans la seconde variante duquel n est égal à 2 et $R^1$ est un groupe $-CH_2-$.

7. Composé suivant la revendication 1, dans sa première variante.

8. Composé de formule stéréochimique

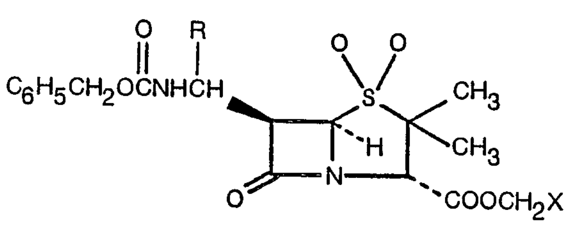

ou

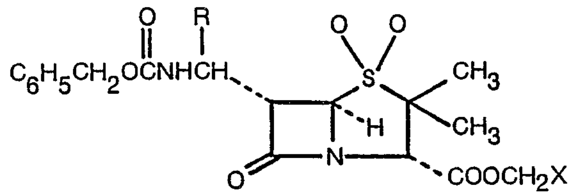

dans laquelle R est l'hydrogène ou le groupe méthyle et X est le radical chloro, ou iodo.

9. Composé de formule stéréochimique

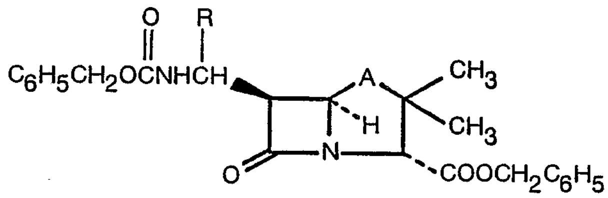

,

ou

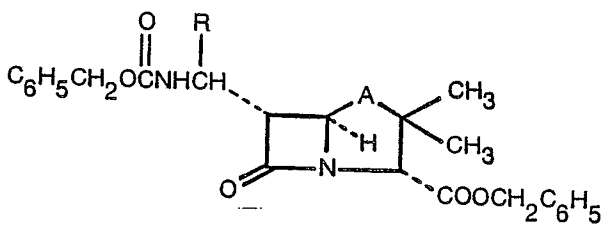

dans laquelle R est l'hydrogène ou le groupe méthyle et A représente

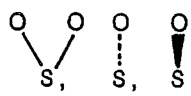

ou S;

10. Composé de formule stéréochimique

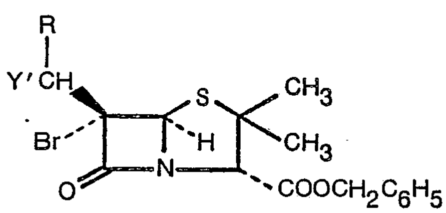

ou

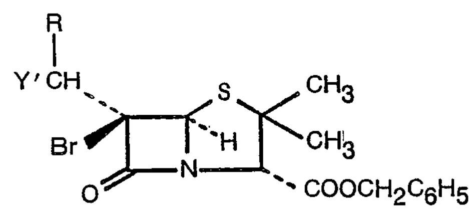

dans laquelle R est l'hydrogène ou le groupe méthyle et Y' est un groupe benzyloxycarbonylamino, amino, azido, ou trifluorométhanesulfonyloxy.

11. Composition pharmaceutique destinée au traitement d'infections bactériennes, qui comprend dans un rapport en poids de 1:3 à 3:1 un composé de la revendication 1 à l'exclusion des composés de la seconde variante dans lesquels Q est l'hydrogène, R est l'hydrogène ou le groupe méthyle, n est égal à 1 et $R^1$ représente $R^4$; et un antibiotique du type bêta-lactame.

12. Composition pharmaceutique suivant la revendication 11, dans laquelle l'antibiotique du type bêta-lactame est le suivant:

amoxicilline,
ampicilline,
azlocilline,
bacampicilline,
carbénicilline,
carbénicilline-indanyle,
carbénicilline-phényle,
céfaclor,

céfadroxile,
céfaloram,
céfamandole,
nafate de céfamandole,
céfaparole,
céfatrizine,
céfazoline,
céfménoxime,
céfonicide,
céfodizime,
céfopérazone,
céforanide,
céfotaxime,
céfoxitine,
céfsulodine,
céftazidime,
céftizoxime,
céftriaxone,
céfuroxime,
céphacetrile,
céphalexine,
céphaloglycine,
céphaloridine,
céphalothine,
céphapirine,
céphradine,
cyclacilline,
épicilline,
hétacilline,
lévopropylcilline,
mécillinam,
mezlocilline,
pénicilline G,
pénicilline V,
phénéthicilline,
pipéracilline,
pirbénicilline,
pivampicilline,
samoxicilline,
sarpicilline,
suncilline,
talampicilline ou
ticarcilline; ou

un sel pharmaceutiquement acceptable de cet antibiotique.

13. Composition pharmaceutique apte à être utilisée dans le traitement d'une infection bactérienne chez un mammifère, qui comprend une quantité efficace du point de vue antibactérien d'un composé suivant la revendication 1 dans sa seconde variante, dans lequel Q est l'hydrogène, R est l'hydrogène ou le groupe méthyle, n est égal à 1 et $R^1$ représente $R^4$, en association avec un diluant ou support pharmaceutiquement acceptable.

**Revendications pour l'Etat contractant: AT**

1. Procédé de préparation d'un composé de formule stéréochimique

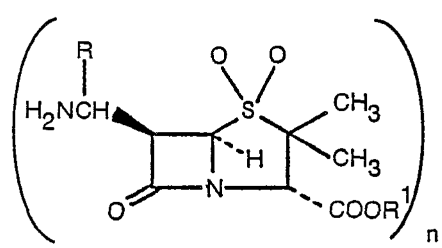 (I) ou 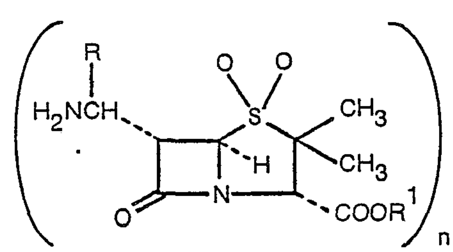 (II)

dans laquelle

R est l'hydrogène ou le groupe méthyle; et

n est égal à 2 et $R^1$ représente $—CH_2—$; ou bien

n est égal à 1 et $R^1$ représente l'hydrogène, un groupe radicalaire formant un ester hydrolysable dans des conditions physiologiques; un groupe 1,1-dioxopénicillanoyloxyméthyle; ou bien un groupe $R^4$, le groupe $R^4$ répondant à la formule

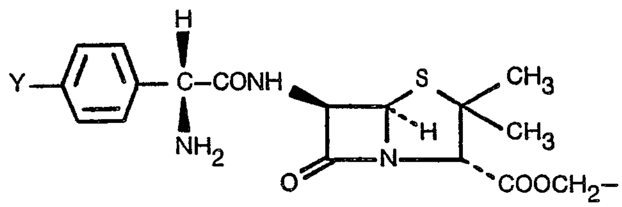

dans laquelle Y représente l'hydrogène, un groupe hydroxy, un groupe alcanoyloxy en $C_2$ à $C_7$, un groupe alkoxycarbonyloxy en $C_2$ à $C_7$, un groupe benzoyloxy ou un groupe benzoyloxy monosubstitué avec un substituant alkyle en C à C, alkoxy en C à C ou halogéno;

qui est caractérisé par l'hydrogénolyse, dans un solvant inert vis-à-vis de la réaction sur un catalyseur à base d'un métal noble, d'un composé de formule

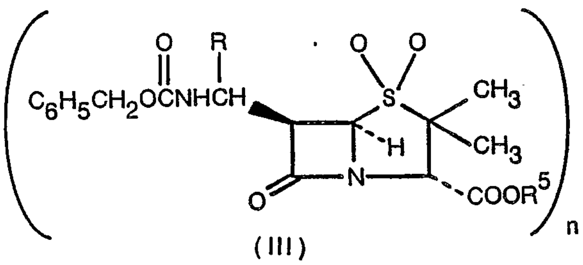

(III)

ou

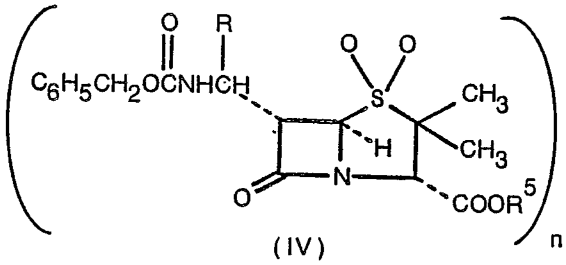

(IV)

dans laquelle

R est l'hydrogène ou le groupe méthyle; et

n est égal à 2 et $R^5$ est le groupe $—CH_2—$; ou bien

n est égal à 1 et $R^5$ est le groupe benzyle, un groupe radicalaire formant un ester hydrolysable dans les conditions physiologiques; un groupe 1,1-dioxopénicillanoyloxyméthyle; ou un groupe de formule

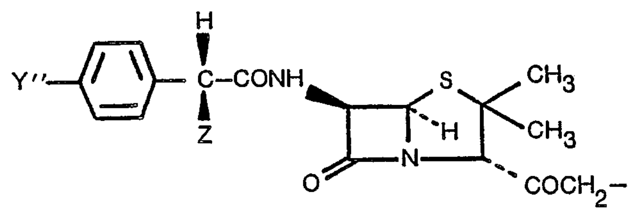

(C)

dans laquelle Y″ est l'hydrogène, un groupe benzyloxycarbonyloxy, un groupe alcanoyloxy en $C_2$ à $C_7$, un groupe alkoxycarbonyloxy en $C_2$ à $C_7$, un groupe benzoyloxy ou un groupe benzoyloxy monosubstitué avec un substituant alkyle en $C_1$ à $C_4$, alkoxy en $C_1$ à $C_4$ ou halogéno; et Z est un groupe azido, ou benzyloxycarbonylamino; et, le cas échéant, transformation dudit composé de formule (I) ou (II) en un sel d'addition d'acide pharmaceutiquement acceptable ou en un sel cationique pharmaceutiquement acceptable lorsque $R^1$ représente l'hydrogène.

2. Procédé suivant la revendication 1, qui est en outre caractérisé par la préparation du composé de formule (III) ou (IV) dans laquelle R est l'hydrogène ou le groupe méthyle, n est é gal à 2 et $R^5$ est le groupe $—CH_2—$ par réaction d'un composé de formule

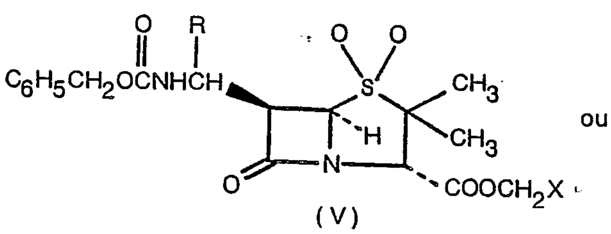

(V)

ou

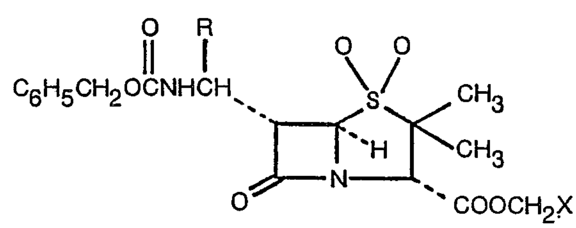

(VI)

dans laquelle R est l'hydrogène ou le groupe méthyle et X est un radical chloro ou bromo, avec un sel d'un acide de formule

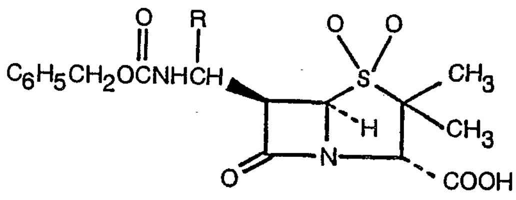

(VII)

ou

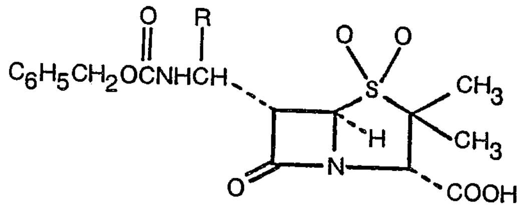

(VIII)

3. Procédé est la revendication 1, qui est en outre caractérisé par la préparation du composé de formule (III) ou (IV) dans laquelle R est l'hydrogène ou le groupe méthyle, n est égal à 1 et $R^5$ est le groupe benzyle, par oxydation en une seule ou plusieurs étapes d'un sulfure de formule

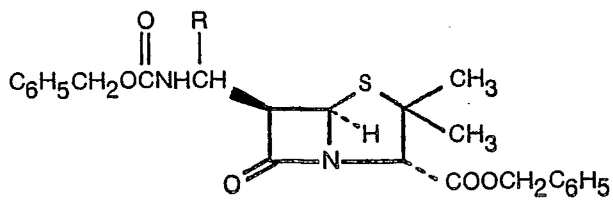

(IX)

ou

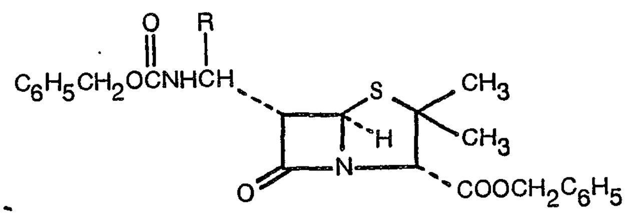

(X)

dans laquelle R est l'hydrogène ou le groupe méthyle.

4. Procédé suivant la revendication 1, caractérisé en outre par la préparation du composé de formule (III) ou (IV) dans laquelle R est l'hydrogène ou le groupe méthyle, n est égal à 1 et $R^5$ est un groupe radicalaire formant un ester hydrolysable dans des conditions physiologiques, choisi entre les groups pivaloyloxyméthyle et acétoxyméthyle, par réaction de pivalate de chlorométhyle ou d'acétate de bromométhyle avec un sel d'un acide de formule (VII) ou (VIII).

5. Procédé suivant la revendication 1, caractérisé en outre par la préparation du composé de formule (III) ou (IV) dans laquelle R est l'hydrogène ou le groupe méthyle, n est égal à 1 et $R^5$ est un groupe 1,1-dioxopénicillanoyloxyméthyle, par réaction de l'ester d'halogénométhyle de formule (V) ou (VI) avec un sel de 1,1-dioxyde d'acide pénicillanique; ou réaction du sel d'un acide de formule (VII) ou (VIII) avec un ester d'halogénométhyle de l'acide pénicillanique.

6. Procédé suivant la revendication 1, caractérisé en outre par la préparation du composé de formule (III) ou (IV) dans laquelle R est l'hydrogène ou le groupe méthyle, n est égal à 1 et $R^5$ est le radical de formule (C) par réaction de l'ester d'iodométhyle de formule (V) ou (VI) avec un sel d'un acide de formule

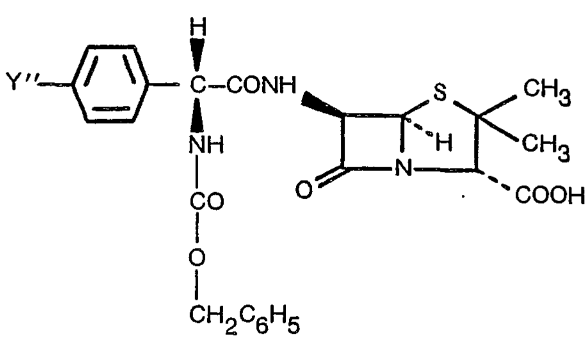

(XI)

dans laquelle Y'' est l'hydrogène, le groupe benzyloxycarbonyloxy, un groupe alkoxycarbonyloxy en $C_2$ à $C_7$, benzoyloxy ou benzoyloxy monosubstitué avec un substituant alkyle en C à C, alkoxy en C à C, ou halogéno; ou réaction du sel de l'acide de formule (VII) ou (VIII) avec un ester d'halogénométhyle de l'acide de formule (XI).

7. Procédé de préparation d'un composé de formule

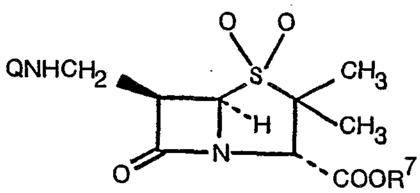 (XII) ou 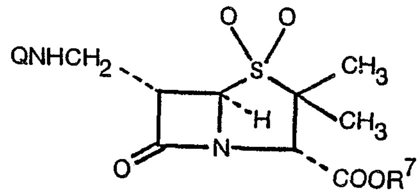 (XIII)

dans laquelle

Q est un groupe benzyle, *o-*, *m-* ou *p*-hydroxybenzyle, phénéthyle, ou 2-, 3- ou 4-picolyle; et

R est l'hydrogène, ou un groupe radicalaire formant un ester qui est hydrolysable dans des conditions physiologiques;

qui est caractérisé par réaction d'un composé aminométhylique de formule

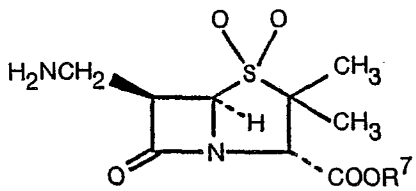 ou 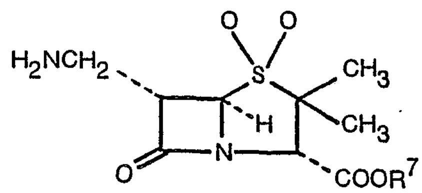

dans laquelle R est tel que défini ci-dessus, avec un équivalent de benzaldéhyde, de *o-*, *m-* ou *p*-hydroxybenzaldéhyde, de phénylacétaldéhyde ou de 2-, 3- ou 4-pyridinecarbaldéhyde en présence de cyanoborohydrure de sodium en excès; et, le cas échéant, transformation dudit composé de formule (XII) ou (XIII) en un sel d'addition d'acide pharmaceutiquement acceptable; ou

transformation d'un composé de formule (XII) ou (XIII) dans laquelle R est l'hydrogène en un sel cationique pharmaceutiquement acceptable.

8. Procédé de préparation d'un composé de formule

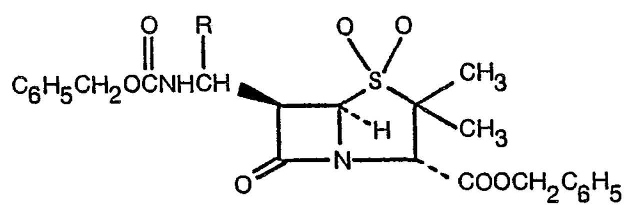

ou

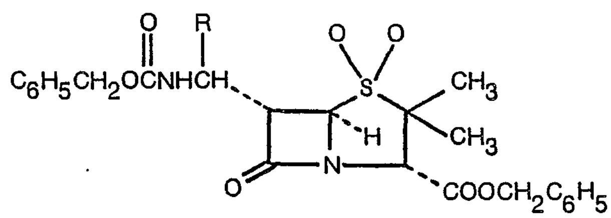

qui est caractérisé par l'oxydation en une seule ou en plusieurs étapes d'un composé de formule

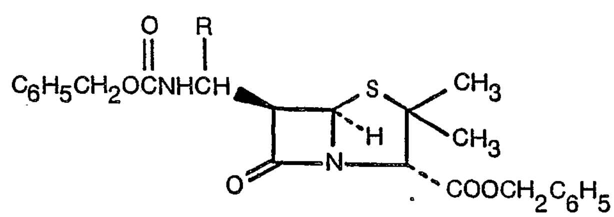

ou

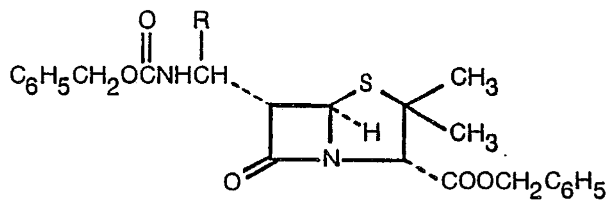

9. Procédé de préparation d'un composé de formule

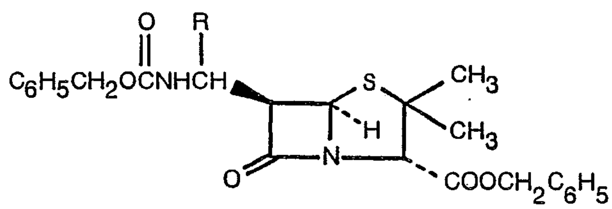

ou

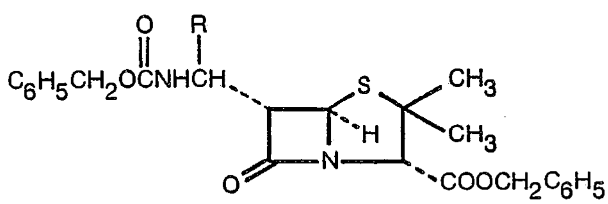

qui est caractérisé par les étapes

(a) de réaction d'un alcool de formule

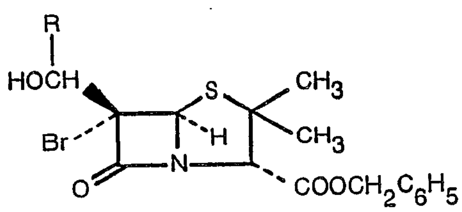

ou

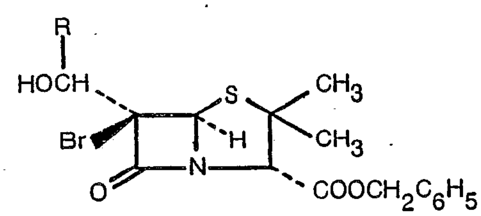

avec le chlorure de trifluorométhanesulfonyle pour former l'ester trifluorométhanesulfonique de formule

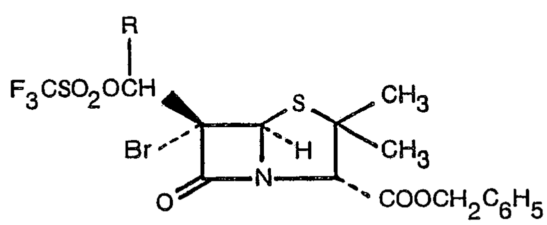

ou

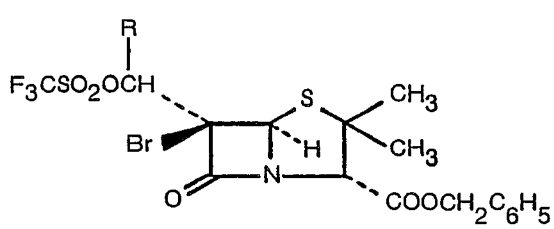

;

(b) de réaction dudit ester trifluorométhanesulfonique avec un sel d'azide pour former un composé azido de formule

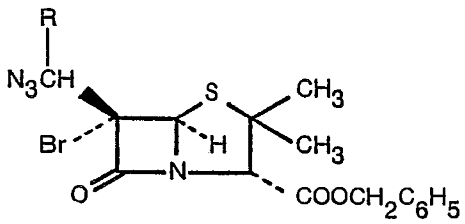

ou

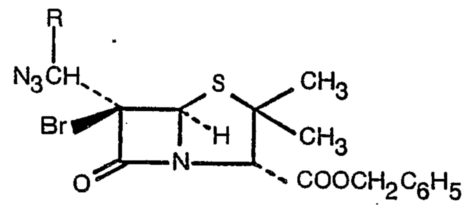

;

c) de réduction dudit composé azido avec le sulfure d'hydrogène pour former un composé amino bromé de formule

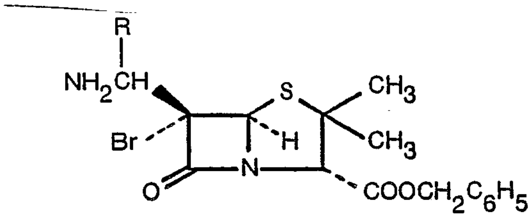

ou

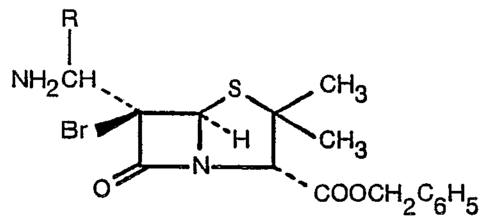

;

(d) de réaction dudit composé amino bromé avec le chloroformiate de benzyle pour former un composé amido bromé de formule

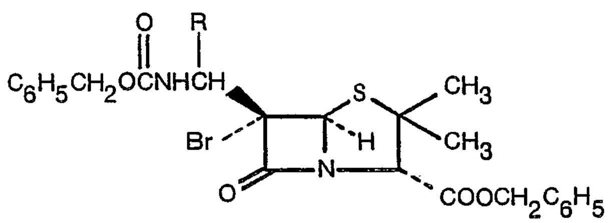

ou

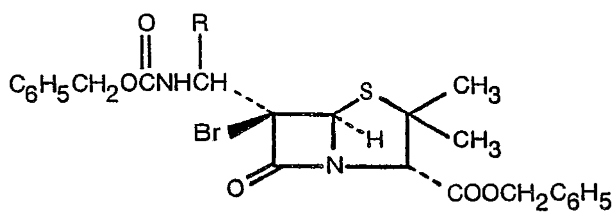

; et

(e) d'élimination du brome dudit composé amido bromé, avec l'hydrure de tri(*n*-butyl)étain.